(19)

**Europäisches Patentamt**

**European Patent Office**

**Office européen des brevets**

(11)  **EP 4 389 886 A1**

(12)  # EUROPEAN PATENT APPLICATION

(43)  Date of publication:
**26.06.2024  Bulletin 2024/26**

(21)  Application number: **22215776.0**

(22)  Date of filing: **22.12.2022**

(51)  International Patent Classification (IPC):
*C12N 9/38* (2006.01)    *A23C 3/03* (2006.01)
*A23C 3/037* (2006.01)   *A23C 9/12* (2006.01)

(52)  Cooperative Patent Classification (CPC):
**A23C 9/1206; A23C 3/03; A23C 3/037;
C12N 9/2471**

---

(84)  Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71)  Applicant: **Novozymes A/S**
**2880 Bagsvaerd (DK)**

(72)  Inventors:
- **SPODSBERG, Nikolaj**
  **2880 Bagsvaerd (DK)**
- **TAMS, Jeppe, Wegener**
  **2880 Bagsvaerd (DK)**
- **RANNES, Julie, Bille**
  **2880 Bagsvaerd (DK)**

- **VESTER, Jan, Kjoelhede**
  **2880 Bagsvaerd (DK)**
- **RASMUSSEN, Frank, Winther,**
  **2880 Bagsvaerd (DK)**
- **JOHANSEN, Annette, Helle**
  **2880 Bagsvaerd (DK)**
- **PACHE, Roland, Alexander**
  **2880 Bagsvaerd (DK)**

(74)  Representative: **NZ EPO Representatives**
**Krogshoejvej 36**
**2880 Bagsvaerd (DK)**

Remarks:
The complete document including Reference Table(s) and the Sequence Listing(s) can be downloaded from the EPO website

---

(54)  ## LACTASE ENZYMES AND METHOD OF PRODUCING A MILK-BASED PRODUCT

(57)    The invention relates to novel enzymes having lactase activity, variants of said enzymes, lactose-reduced milk-based products comprising the lactase enzymes, and methods of producing a lactose-reduced, heat-treated, milk-based product using the lactase enzymes.

**EP 4 389 886 A1**

**Description**

**Reference to sequence listing**

This application contains a Sequence Listing in computer readable form. The computer readable form is incorporated herein by reference.

## FIELD OF THE INVENTION

The present invention relates to novel enzymes having lactase activity, variants of said enzymes, a method of producing a lactose-reduced, heat-treated, milk-based product using the lactase enzymes, and lactose-reduced milk-based products comprising the lactase enzymes.

## BACKGROUND OF THE INVENTION

Most lactose-reduced or lactose-free milk-based products are produced in batch processes, i.e. adding lactase to milk and then incubating the milk at cold temperatures (usually less than 10°C) for enough time to reduce the lactose content to less than 0.01% or less than 0.1% (which in most countries allows for labelling of, e.g., milk as lactose-free), followed by heat treatment such as pasteurization, UHT or ESL (extended shelf-life of up to 35 days) treatments. Some of the drawbacks linked to the batch application of lactases include:

(1) the incubation time can be up to 24 h and sometimes more, which raises concerns regarding capacity and capital investments in new incubation tanks to respond to the growing demand for lactose free dairy products.

(2) Prolonged incubation times give psychotropic microbes a chance to grow and secrete their enzymes (particularly proteases), some of which are heat stable and may deteriorate the quality of the final product during the shelf-life.

(3) Lactose hydrolysis followed by severe heat treatment, such as in UHT applications, will result in Maillard reaction being propagated at an accelerated rate. When lactose is hydrolyzed to glucose and galactose, the concentration of sugars having reducing ends is doubled and especially galactose is much more reactive than lactose.

Recently and specifically in the case of lactose-free ESL and UHT milk drinks, advanced process engineering technologies in the form of aseptic dosing equipment have gained a lot of interest and made it possible to overcome many of the concerns linked to the batch process. Such aseptic dosing equipment makes it possible to add the lactase after the ESL/UHT treatment. Examples of such aseptic dosing equipment are the Tetra Pak® Aldose system, Tetra Pak® Flexdose System, and GEA Varidose system. The advantages of these systems are:

(1) Dosing smaller quantities of sterile lactases with high precision into the milk stream after the heat treatment step, allowing lactose hydrolysis to take place during the first few days of storage.

(2) Decreasing the extent of Maillard reaction, browning and the formation of Advanced Gly-cation End products (AGEs) in lactose-free milk drinks, especially when proper controlled storage conditions are applied.

(3) Omitting the pre-incubation step compared to the batch process and thus solving the capacity issues and decreasing the risk of activity of psychotropic microbes.

Despite the above advantages of aseptic dosing systems, there are a number of drawbacks associated with their use, such as:

(1) Since the lactases are added after the heat treatment step, their formulation should be of the highest possible purity. This is because any detrimental side activity in their formulation may have very negative impact on the end product during the long shelf-life, especially in the case of UHT products.

(2) In the case of Tetra Pak® Flexdose and GEA Varidose systems, the lactase enzyme should be aseptically filled in sterile buckets and bags.

(3) The above requirements in points (1) and (2) mean these sterile lactases cost more per unit of activity compared to lactases used in the batch process.

(4) The average capital cost of these aseptic dosing systems is substantial.

(5) There are additional running costs linked to the consumables when using Tetra Pak® Flexdose and GEA Varidose systems. An example of this is changing the hose and needle every time an aseptic bucket is changed.

(6) In the Tetra Pak® Aldose system, the enzyme formulation is not initially sterile. It is diluted with water and then filtered in line (in the dairy) using at least two filters to ensure sterility of the enzyme stream before mixing it with the milk stream. This can be problematic, as the filters installed in-line may become blocked by poorly filterable enzyme formulation, which may cause difficulties during operation. Even when the filterability of the enzyme formulation works as expected, there is still a need to change the filters regularly (usually daily).

A quick, smooth, easy to implement, trouble-free, cost-effective solution for using lactases in lactose-free UHT and ESL products, which overcomes all the above limitations of both batch and aseptic dosing processes, does not yet exist.

WO2009/071539 (Novozymes) relates to a method of producing a dairy product using an enzyme having lactase activity. Disclosed is a method of producing a low-lactose milk product by treating a milk-based substrate with lactase at high temperature, i.e., at least 60°C, at least 62°C, at least 63°C, at least 64°C, at least 65°C, at least 67°C, at least 70°C, or at least 75°C.

WO2018/189238 (Chr. Hansen) discloses beta-galactosidases which are said to be stable with relatively high activity at a broad range of temperatures and pH values. Disclosed is a method for producing a dairy product by treating a milk-based substrate with a beta-galactosidase, wherein the treatment or part of the treatment may take place at high temperature. A lactose concentration of less than 0.2% lactose may be obtained in 3-30 minutes after adding the beta-galactosidase.

WO2020/176734 (DuPont) relates to a method for reducing the amount of lactose in a milk-based substrate by contacting the substrate with a lactase, such as a thermostable lactase, at high temperature. Disclosed is a method for production of a lactose free dairy product from a milk-based substrate with an enzyme having neutral lactase activity wherein more than 20% lactase activity remains in the milk-based substrate after pasteurization at 72°C for 15 seconds. Such pasteurization is also sometimes referred to as high-temperature, short-time (HTST) pasteurization.

Deeth (2017) "Optimum Thermal Processing for Extended Shelf-Life (ESL) Milk", Foods 6(11): 102 has reviewed the optimum thermal processing for Extended Shelf-Life (ESL) milk. Deeth explains that ESL or ultra-pasteurized milk is produced by thermal processing using conditions between those used for traditional high-temperature, short-time (HTST) pasteurization and those used for ultra-high-temperature (UHT) sterilization. ESL milk should have a refrigerated shelf-life of more than 30 days. To achieve this, the thermal processing must be quite intense. Unlike the temperature-time conditions for pasteurization, which are specified in most countries to be at least 72°C for at least 15 s, there are generally no such specified conditions for ESL processing. According to Deeth (2017), reported commercial processing conditions for ESL milk are mostly in the range of 123-127°C for 1-5 seconds. U.S. regulations define the process of "ultra-pasteurization" as heating milk at a temperature of at least 138°C for at least 2 seconds.

European patent application No. 21216998.1 discloses a method of producing lactose-reduced, heat-treated, milk-based product, e.g. milk, using an enzyme having lactase activity without the need to perform an extensive pre-incubation of the milk-based substrate with the enzyme and without the need to use aseptic dosing systems to add the enzyme after the heat treatment. In this method, after the heat-treatment, such as a UHT treatment, the enzyme has some residual activity which ensures lactose degradation to the desired low lactose level during storage in the cold or at ambient temperature.

UHT treatment may be, e.g., heat treatment for 30 seconds at 130°C, for 3-4 seconds at 140°C or for 1 second at 145°C.

There is still a need for improved methods for producing lactose-reduced, heat-treated, milk-based products such as UHT or ESL milk, as well as for lactase enzymes that are suitable for such methods.

## SUMMARY OF THE INVENTION

The invention provides novel enzymes having lactase activity as well as variants thereof, as well as milk-based products comprising such enzymes, where the lactase enzymes have sufficient residual enzymatic activity after heat treatment, such as UHT or ESL treatment, for production of lactose-reduced milk-based products. This avoids the need to perform an extensive pre-incubation of the milk-based substrate with the enzyme, as well as the need to use aseptic dosing systems to add the enzyme after the heat treatment. The invention further provides methods for producing milk-based products using the lactase enzymes.

One aspect of the invention thus relates to a milk-based product comprising an enzyme having lactase activity, wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

Another aspect of the invention relates to the lactase enzymes themselves as well as variants thereof.

Another aspect of the invention relates to a method of producing a lactose-reduced, heat-treated, milk-based product, the method comprising:

a) adding an enzyme having lactase activity to a milk-based substrate comprising at least 2% lactose (w/w), wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13,

b) after addition of the enzyme, performing a heat treatment of the milk-based substrate by holding said milk-based substrate at a holding temperature of at least 120°C for a holding time of at least 1 second followed by cooling to produce a heat-treated milk-based product, and

c) storing the heat-treated milk-based product for at least about 24 hours, preferably at least 2 days, such as at least 3 days, preferably at least 4 days at a temperature of at most 40°C,

wherein after step c) the lactose content in the milk-based product is at most 0.2% (w/w).

Preferably, after step b) but before step c), the lactose content in the milk-based product is at least 0.5% (w/w), preferably at least 1% (w/w).

A further aspect of the invention relates to a method of producing a lactose-reduced milk product, the method comprising adding an enzyme having lactase activity to a milk-based substrate comprising at least 2% lactose (w/w), and subjecting the milk-based substrate comprising the enzyme to a heat treatment at a temperature of at least 120°C for a holding time of at least 1 second, wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

A still further aspect of the invention relates to use of an enzyme having lactase activity for producing a lactose-reduced, heat-treated, milk-based product in a method wherein a milk-based substrate comprising at least 2% lactose (w/w) to which the enzyme has been added is subjected to a heat treatment at a temperature of at least 120°C for a holding time of at least 1 second, wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

Preferably, the enzyme having lactase activity is added immediately before the heat-treatment, such as the UHT treatment. After the heat-treatment, such as the UHT treatment, the enzyme has some residual activity which ensures lactose degradation to the desired low lactose level during storage in the cold or at ambient temperature. Preferably, the enzyme has a temperature optimum of 30-60°C, more preferably 35-55°C. Without wishing to be bound by theory, it is believed that an enzyme having a higher temperature optimum and perhaps even being active during the heat treatment will have a somewhat rigid structure and will not have sufficient activity during storage in the cold or at ambient temperature. Again without wishing to be bound by theory, it is believed that an enzyme having a temperature optimum of 30-60°C, more preferably 35-55°C, to be used in the method of the invention may unfold and be inactive during the heat treatment but have the ability to refold and be reactivated afterwards and therefore have a measurable or even substantial residual activity, ensuring lactose degradation during storage of the milk-based product. It is contemplated that the high residual activity of the lactase enzymes of the invention after heat treatment such as UHT treatment may be due to an ability to refold and become reactivated once the temperature is lowered.

Preferably, the enzyme has a residual activity of at least 1%, preferably at least 2%, more preferably at least 5%, even more preferably at least 10%, after incubation at 70°C for 30 seconds and 140°C for 5 seconds in skimmed milk having a lactose content of 4.7%.

More preferably, the enzyme has a residual activity of at least 0.5%, preferably at least 1%, at least 2% or at least 3%, more preferably at least 5%, even more preferably at least 10%, after incubation in skimmed milk having a lactose content of 4.7% at 90°C for 30 seconds, at 140°C for 5 seconds and at 70°C for 30 seconds followed by cooling to 0-10°C and subsequent incubation at 23°C for 72 hours, wherein the residual activity is relative to the activity of the same enzyme in skimmed milk without incubation at 90°C for 30 seconds, at 140°C for 5 seconds and at 70°C for 30 seconds followed by cooling to 0-10°C and subsequent incubation at 23°C for 72 hours.

Preferably, the milk-based substrate is not incubated with the lactase before the heat treatment except for the time it takes - depending on the process equipment - from addition of the lactase to the milk-based substrate has reached the holding temperature of the heat treatment. Preferably, step b) is performed immediately after step a) without a dedicated incubation step between step a) and step b).

At least three main process options can be used according to the method:

In a first option, the milk-based substrate may be mixed with the lactase, and then processed directly under UHT or ESL conditions without the need to incubate the milk-based substrate with the lactase.

In a second option, the milk-based substrate may be processed directly under UHT or ESL conditions where the lactase is added to the milk-based substrate while said milk-based substrate is streaming through process pipes immediately before the heat treatment step, optionally while the temperature of the milk-based substrate is increasing towards the temperature of the heat treatment step. The lactase may be added at any point where the temperature of the milk-based substrate is 1-95°C, preferably 70-90°C, just before the UHT/ESL treatment. The addition of the lactase may take place via a simple dosing pump through a tube which is connected to the main milk stream tube. Once added to the running stream of milk-based substrate, the temperature is (further) raised to the ESL or UHT processing conditions. A heating medium may be used which is not in direct contact with the milk-based substrate but separated by equipment contact surfaces, such as a plate heat-exchanger or a tubular heat-exchanger. This may be referred to as an indirect heat treatment, preferably an indirect UHT treatment.

In a third option, the heat treatment is performed by steam injection or steam infusion, preferably steam injection, using high-pressure steam to heat the milk-based substrate, and the enzyme may be added with the steam. This may be referred to as a direct heat treatment, preferably a direct UHT treatment. After the holding time of step b), the milk-based substrate comprising the steam may be flash-cooled in a vacuum to remove water equivalent to the amount of condensed steam used. In addition to the heating of the milk-based substrate by steam injection or steam infusion, indirect heating may also be applied, e.g., using a plate or tubular heat exchanger.

In any case, the residual activity of the lactase after the heat treatment ensures that lactose levels will decrease to lactose-reduced, preferably lactose-free levels (e.g., less than 0.1% or less than 0.01 %) during the initial stage of storage (e.g., up to 2 weeks, such as during the first 2 or 3 days).

The method of the invention has a number of advantages over the processes used today for production of ESL- and UHT-treated milk-based products, such as ESL or UHT milk.

Compared to the batch processes applied today, the process of the invention provides an improved color and quality of the lactose-reduced or lactose free milk-based product due to reduced Maillard reaction. Without preincubation, there will be reduced growth of psychotropic bacteria secreting enzymes including proteases possibly surviving the heat treatment, and therefore the milk-based product can have a longer shelf life. Further, without preincubation, capacity cost and process time is reduced. The method of the invention is also easier to operate since there is no need to monitor the tanks until the lactose level is below 0.1% or 0.01%.

Compared to the aseptic dosing processes applied today, in the method using the lactase enzymes of the invention there is no need to invest in the aseptic dosing equipment and no need for, e.g., regular changing of the aseptic bucket. Further, the currently available sterile lactases have a shelf life of approximately one year, while the lactase enzymes used in the method of the invention can have a longer shelf life of two years or more. The method of the invention is easy to operate and hassle-free as there is no need to monitor or troubleshoot any of the aseptic dosing systems. The final quality of the milk-based product is the same regarding color and Maillard reaction.

The method using the lactases of the invention is easy to apply on an industrial scale and there is no barrier for its implementation.

## DRAWING DESCRIPTION

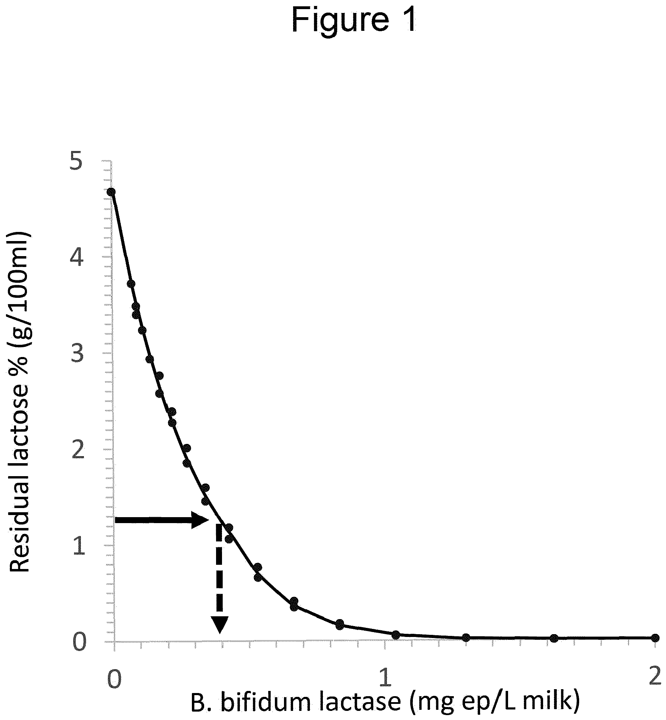

Figure 1 shows an example of a dose-response curve depicting residual lactose content in milk as a function of lactase concentration.

## OVERVIEW OF SEQUENCES

SEQ ID NO: 1 is a C-terminally truncated lactase from *Bifidobacterium samirii.*

SEQ ID NO: 2 is a C-terminally truncated lactase from *Streptococcus entericus DSM 14446.*

SEQ ID NO: 3 is a C-terminally truncated lactase from *Varibaculum sp.*

SEQ ID NO: 4 is a C-terminally truncated lactase from *Urmitella timonensis*

SEQ ID NO: 5 is a C-terminally truncated lactase from *Bifidobacterium bifidum.*

SEQ ID NO: 6 is a C-terminally truncated lactase from *Bacillus sp. S3.*

SEQ ID NO: 7 is a C-terminally truncated lactase from *Bifidobacterium aerophilum.*

SEQ ID NO: 8 is a C-terminally truncated lactase from *Bifidobacterium* mongoliense.

SEQ ID NO: 9 is a C-terminally truncated lactase from *Clostridium nexile CAG:348.*

SEQ ID NO: 10 is a C-terminally truncated lactase from *Neobacillus bataviensis.*

SEQ ID NO: 11 is a C-terminally truncated lactase from *Neobacillus mesonae.*

SEQ ID NO: 12 is a C-terminally truncated lactase from *Neobacillus niacin.*

SEQ ID NO: 13 is a C-terminally truncated lactase from *Streptomyces cirratus.*

SEQ ID NO: 14 is a secretion signal from *Bacillus clausii.*

SEQ ID NO: 15 is a poly histidine tag.

## DEFINITIONS

In accordance with this detailed description, the following definitions apply. Note that the singular forms "a," "an," and "the" include plural references unless the context clearly dictates otherwise.

Unless defined otherwise or clearly indicated by context, all technical and scientific terms used herein have the same meaning as commonly understood by one of ordinary skill in the art to which this invention belongs.

**Isolated:** The term "isolated" means a polypeptide, nucleic acid, cell, or other specified material or component that has been separated from at least one other material or component, including but not limited to, other proteins, nucleic acids, cells, etc. An isolated polypeptide, nucleic acid, cell or other material is thus in a form that does not occur in nature. An isolated polypeptide includes, but is not limited to, a culture broth containing the secreted polypeptide expressed in a host cell.

**Lactase:** The term "lactase" means a glycoside hydrolase having the ability to hydrolyze the disaccharide lactose into constituent galactose and glucose monomers. The group of lactases comprises but is not limited to enzymes assigned to subclass EC 3.2.1.108. Enzymes assigned to other subclasses, such as, e.g., EC 3.2.1.23 or EC 3.2.1.21, may also be lactases in the context of the present invention. A lactase in the context of the invention may have other activities than the lactose hydrolyzing activity, such as for example a transgalactosylating activity. In the context of the invention, the lactose hydrolyzing activity of the lactase may be referred to as its lactase activity, its beta-galactosidase activity or its hydrolyzing activity.

**Lactase Activity:** Lactase activity may be determined using, e.g., a LAU(B) assay. The activity in LAU(B) of a specific lactase may be determined by direct measurement of o-nitrophenyl (ONP) released from o-nitrophenyl β-D-galactopyranoside (ONPG) in a buffer containing 1.46 mg/ml substrate in 0.05 M MES, 1 mM MgSO4 7H2O, 450 mg/L Brij 35 at pH6.5 and 30°C. After 600 seconds incubation, the reaction is stopped by adding 0.2 M Na2CO3 and the released ONP is measured at 405 nm after 126 seconds incubation. The activity is obtained by comparing to a standard curve run with a lactase of known activity, and the activity of the unknown sample calculated from this. The lactase of known activity may, e.g., be Saphera®, available from Novozymes A/S, Denmark. Lactase activity may also be determined by measuring the amount of lactose hydrolysis in milk, e.g. by the method described in Example 1 in the paragraph "Analysis of residual lactose content" using HPAEC-PAD where the lactose peak is related to a lactose standard with known concentration. The lactose hydrolysis can then be related to the amount of lactase added, e.g. per mg enzyme protein or per mole enzyme. Other methods for measuring lactase activity are known and used routinely in the art.

**Mature polypeptide:** The term "mature polypeptide" means a polypeptide in its mature form following N terminal and/or C-terminal processing (e.g., removal of signal peptide).

**Milk:** The term "milk" means the lacteal secretion obtained by milking any mammal, such as cows, sheep, goats, buffaloes or camels.

**Milk-based product:** The term "milk-based product" refers to milk products and other products based on milk.

In the present context it will be apparent that the term includes in particular heat-treated milk-based products, including not only pasteurized milk but also milk that is subjected to higher temperatures than those typically used in pasteurization, such as ultra-pasteurized milk, UHT (ultra-high temperature) milk and ESL (extended shelf life) milk.

**Purified:** The term "purified" means a nucleic acid, polypeptide or cell that is substantially free from other components as determined by analytical techniques well known in the art (e.g., a purified polypeptide or nucleic acid may form a discrete band in an electrophoretic gel, chromatographic eluate, and/or a media subjected to density gradient centrifugation). A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight or on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

In one aspect, the term "purified" as used herein refers to the polypeptide or cell being essentially free from components (especially insoluble components) from the production organism. In other aspects. the term "purified" refers to the polypeptide being essentially free of insoluble components (especially insoluble components) from the native organism from which it is obtained. In one aspect, the polypeptide is separated from some of the soluble components of the organism and culture medium from which it is recovered. The polypeptide may be purified (i.e., separated) by one or more of the unit operations filtration, precipitation, or chromatography.

Accordingly, the polypeptide may be purified such that only minor amounts of other proteins, in particular, other polypeptides, are present. The term "purified" as used herein may refer to removal of other components, particularly other proteins and most particularly other enzymes present in the cell of origin of the polypeptide. The polypeptide may be "substantially pure", i.e., free from other components from the organism in which it is produced, e.g., a host organism for recombinantly produced polypeptide. In one aspect, the polypeptide is at least 40% pure by weight of the total polypeptide material present in the preparation. In one aspect, the polypeptide is at least 50%, 60%, 70%, 80% or 90% pure by weight of the total polypeptide material present in the preparation. As used herein. a "substantially pure polypeptide" may denote a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which the polypeptide is natively or recombinantly associated.

It is, therefore, preferred that the substantially pure polypeptide is at least 92% pure, preferably at least 94% pure, more preferably at least 95% pure, more preferably at least 96% pure, more preferably at least 97% pure, more preferably at least 98% pure, even more preferably at least 99% pure, most preferably at least 99.5% pure by weight of the total polypeptide material present in the preparation. The polypeptide of the present invention is preferably in a substantially pure form (i.e., the preparation is essentially free of other polypeptide material with which it is natively or recombinantly associated). This can be accomplished, for example by preparing the polypeptide by well-known recombinant methods or by classical purification methods.

**Sequence identity:** The relatedness between two amino acid sequences or between two nucleotide sequences is described by the parameter "sequence identity".

For purposes of the present invention, the sequence identity between two amino acid sequences is determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the nobrief option must be specified in the command line. The output of Needle labeled "longest identity" is calculated as follows:

$$\text{(Identical Residues} \times 100)/(\text{Length of Alignment} - \text{Total Number of Gaps in Alignment)}$$

**Variant:** The term "variant" means a polypeptide having lactase activity comprising a man-made mutation, i.e., a substitution, insertion (including extension), and/or deletion (e.g., truncation), at one or more positions. A substitution means replacement of the amino acid occupying a position with a different amino acid; a deletion means removal of the amino acid occupying a position; and an insertion means adding 1-5 amino acids (e.g., 1-3 amino acids, in particular 1 amino acid) adjacent to and immediately following the amino acid occupying a position.

**Wild-type:** The term "wild-type" in reference to an amino acid sequence or nucleic acid sequence means that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence). As explained below, however, the enzymes of the invention having lactase activity have preferably been C-terminally truncated compared to the full-length wild-type enzymes from which they are obtained, i.e. truncated compared to the full-length amino acid sequences encoded by the full genomic DNA sequences.

**Conventions for Designation of Variants**

For purposes of the present invention, a polypeptide having a chosen wild-type sequence may be used to determine the corresponding amino acid positions in another lactase. The amino acid sequence of another lactase is aligned with the polypeptide having a chosen wild-type sequence, and based on the alignment, the amino acid position number corresponding to any amino acid residue in the polypeptide having a chosen wild-type sequence is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

In describing the variants of the present invention, the nomenclature described below is adapted for ease of reference. The accepted IUPAC single letter amino acid abbreviation is employed. The amino acid numbering of the variants disclosed herein is in each case based on the numbering of the relevant wild-type sequence.

For an amino acid substitution, the following nomenclature is used: Original amino acid, position, substituted amino acid. Accordingly, the substitution of threonine at position 226 with alanine is designated as "T226A". Multiple mutations are separated by addition marks ("+"), a comma or simply by a space, e.g., substitutions at positions 205 and 411 of glycine (G) with arginine (R) and serine (S) with phenylalanine (F) may be represented by "G205R + S411 F" or "G205R S411F".

**DETAILED DESCRIPTION OF THE INVENTION**

As mentioned above, the invention provides a milk-based product comprising an enzyme having lactase activity, wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13. Preferably, the enzyme has at least 70%, at least 75% or at least 80% sequence identity to any of said sequences.

The invention also provides an enzyme having lactase activity, wherein the enzyme having lactase activity comprises an amino acid sequence having at least 60% sequence identity, such as at least 70%, at least 75% or at least 80%, to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

Preferably, the milk-based product is one which has been heat-treated and has a lactose content of at most 0.2% (w/w). The lactose content may e.g. be at most 0.1% or at most 0.01 % (w/w).

Preferably, the milk-based product is UHT milk, ESL milk or ultra-pasteurized milk.

The enzyme having lactase activity may, for example, comprise an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13, or to a mature polypeptide of any of SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In one preferred embodiment, the enzyme having lactase activity comprises an amino acid sequence having at least 70% sequence identity, preferably at least 75% sequence identity, such as at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4, or to a mature polypeptide of any of SEQ ID NO: 1, SEQ ID NO: 2, SEQ ID NO: 3 or SEQ ID NO: 4.

The enzyme having lactase activity may e.g. be a variant of any of SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 comprising one or more alterations, e.g., substitutions, deletions and/or insertions, typically substitutions. In preferred embodiments, the enzyme is a variant of SEQ ID NO: 1, SEQ ID NO: 2, SEQ ID NO: 3 or SEQ ID NO: 4. In some more preferred embodiments, the enzyme is a variant of SEQ ID NO: 1.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 1.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 2.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 3.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 4.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 6.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 7.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 8.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 9.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 10.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 11.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 12.

In one embodiment, the enzyme having lactase activity has an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 13.

In some preferred embodiments, the enzyme having lactase activity is a polypeptide based on any of SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13, or on a mature polypeptide of any of SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13, having 1-30 alterations, e.g., substitutions, deletions and/or insertions at one or more positions, e.g., 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 alterations, in particular substitutions. In the present context, "based on" means that the enzyme may be derived from any of the listed wild-type sequences with one or more alterations, and/or may be an enzyme having a longer amino acid sequence than one of the listed wild-type sequences (within the preferred limits on sequence length that are disclosed elsewhere herein), for example by way of a C-terminal extension.

In one embodiment, the enzyme having lactase activity is a polypeptide derived from or based on SEQ ID NO: 1 or from a mature polypeptide of SEQ ID NO: 1, having 1-30 alterations, e.g., substitutions, deletions and/or insertions, typically substitutions, at one or more positions, for example 1-20 alterations, such as 1-15 alterations.

In a preferred embodiment, the enzyme having lactase activity is a variant of SEQ ID NO: 1 having one or more substitutions selected from the group consisting of P65A, C372A, P615T, A1073C, H1122C and C1195G, for example two, three, four or more of said substitutions. Some non-limiting examples of such combinations of substitutions in SEQ ID NO: 1 are A1073C+H1122C, C372A+A1073C+H1122C, C372A+C1195G, A1073C+H1122C+C1195G and C372A+A1073C+ H1122C+C1195G. The variant of this embodiment may have an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 1.

In one embodiment, the enzyme having lactase activity is a polypeptide derived from or based on SEQ ID NO: 2 or from a mature polypeptide of SEQ ID NO: 2, having 1-30 alterations, e.g., substitutions, deletions and/or insertions, typically substitutions, at one or more positions, for example 1-20 alterations, such as 1-15 alterations.

In a preferred embodiment, the enzyme having lactase activity is a variant of SEQ ID NO: 2 having one or more substitutions selected from the group consisting of P52A, G607T, L1064C and Y1110C, for example two or more of said substitutions such as L1064C+Y1110C. The variant of this embodiment may have an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 2.

In one embodiment, the enzyme having lactase activity is a polypeptide derived from or based on SEQ ID NO: 3 or from a mature polypeptide of SEQ ID NO: 3, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 4 or from a mature polypeptide of SEQ ID NO: 4, having 1-30 alterations, e.g., substitutions, deletions and/or insertions, typically substitutions, at one or more positions, for example 1-20 alterations, such as 1-15 alterations.

In a preferred embodiment, the enzyme having lactase activity is a variant of SEQ ID NO: 4 having the substitution G386Q and/or P620T. The variant of this embodiment may have an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 4.

In one embodiment, the enzyme having lactase activity is a polypeptide derived from or based on SEQ ID NO: 6 or from a mature polypeptide of SEQ ID NO: 6, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 7 or from a mature polypeptide of SEQ ID NO: 7, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 8 or from a mature polypeptide of SEQ ID NO: 8, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 9 or from a mature polypeptide of SEQ ID NO: 9, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 10 or from a mature polypeptide of SEQ ID NO: 10, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 11 or from a mature polypeptide of SEQ ID NO: 11, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 12 or from a mature polypeptide of SEQ ID NO: 12, having 1-30 alterations, e.g., substitutions, deletions and/or insertions, typically substitutions, at one or more positions, for example 1-20 alterations, such as 1-15 alterations.

In one embodiment, the enzyme having lactase activity is a polypeptide derived from or based on SEQ ID NO: 13 or from a mature polypeptide of SEQ ID NO: 13, having 1-30 alterations, e.g., substitutions, deletions and/or insertions, typically substitutions, at one or more positions, for example 1-20 alterations, such as 1-15 alterations.

In one embodiment, the number of amino acid substitutions, deletions and/or insertions introduced into the polypeptide of any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 is up to 15, e.g., 1, 2, 3, 4, 5, 6, 7, 8, 9, 10, 11, 12, 13, 14, or 15.

The amino acid changes may be of a minor nature, that is conservative amino acid substitutions or insertions that do not significantly affect the folding and/or activity of the protein; small deletions, typically of 1-30 amino acids; small amino or carboxyl-terminal extensions, such as an amino-terminal methionine residue; a small linker peptide of up to 20-25 residues; or a small extension that facilitates purification by changing net charge or another function, such as a poly-histidine tag, an antigenic epitope or a binding module.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for lactase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide, and/or be inferred from sequence homology and conserved catalytic machinery with a related polypeptide or within a polypeptide or protein family with polypeptides/proteins descending from a common ancestor, typically having similar three-dimensional structures, functions, and significant sequence similarity. Additionally or alternatively, protein structure prediction tools can be used for protein structure modelling to identify essential amino acids and/or active sites of polypeptides. See, for example, Jumper et al., 2021, "Highly accurate protein structure prediction with AlphaFold", Nature 596: 583-589.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Low-man et al., 1991, Biochemistry 30: 10832-10837; US 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Amino acid alterations may also be non-conservative alterations, for example non-conservative substitutions, deletions and/or insertions, typically substitutions, which provide the enzyme with one or more desired characteristics. Non-limiting examples of such desired characteristics include improved performance, increased residual activity after being subjected to elevated temperature, increased refolding ability after denaturation, increased specific activity and reduced oxidation sensitivity.

In a preferred embodiment, the enzyme having lactase activity is derived from or based on any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 by substitution of 1-30, preferably 1-20, amino acids, so that the enzyme has a higher residual activity after a UHT treatment compared to the wildtype enzyme from which it is derived.

Preferably, the enzyme having lactase activity is a bacterial enzyme, i.e. obtained from a bacterial source or a variant of an enzyme obtained from a bacterial source.

In a preferred embodiment, the enzyme having lactase activity is obtained from *Bifidobacterium,* preferably from *Bifidobacterium samirii, Bifidobacterium aerophilum or Bifidobacterium mongoliense,* or from *Bacillus,* preferably from *Bacillus sp. S3,* from *Varibaculum sp.,* from *Urmitella,* preferably from *Urmitella timonensis,* from *Streptococcus,* preferably from *Streptococcus entericus DSM 14446* or *Streptomyces cirratus,* from *Clostridium,* preferably from *Clostridium nexile CAG:348,* or from *Neobacillus,* preferably from *Neobacillus bataviensis, Neobacillus mesonae* or *Neobacillus niacini,* or is a variant of an enzyme obtained from the above-listed genera or species.

The enzymes may be identified and obtained from other sources including microorganisms isolated from nature (e.g., soil, composts, water, etc.) or DNA samples obtained directly from natural materials (e.g., soil, composts, water, etc.) using the above-mentioned probes. Techniques for isolating microorganisms and DNA directly from natural habitats are well known in the art. A polynucleotide encoding the enzyme may then be obtained by similarly screening a genomic DNA or cDNA library of another microorganism or mixed DNA sample. Once a polynucleotide encoding an enzyme has been detected with the probe(s), the polynucleotide can be isolated or cloned by utilizing techniques that are known to those of ordinary skill in the art (see, e.g., Davis et al., 2012, Basic Methods in Molecular Biology, Elsevier).

The enzyme having lactase activity may be a polypeptide derived from any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 or from a mature polypeptide of any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 wherein the N- and/or C-terminal end has been extended by addition of one or more amino acids or wherein one or more amino acids have been deleted from the N- and/or the C-terminal.

The enzyme having lactase activity has preferably been C-terminally truncated compared to the full-length wild-type enzyme from which it is obtained, i.e. the enzyme encoded by the genomic DNA sequence.

The enzyme of the invention having lactase activity may thus be a polypeptide derived from any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13 or from a mature polypeptide of any of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12, wherein the polypeptide has a length of at the most about 1500 amino acids, such as at the most about 1400 amino acids or at the most about 1350 amino acids, for example 850-1500 amino acids, preferably 850-1400 amino acids, such as 850-1350 amino acids; or derived from SEQ ID NO: 13, wherein the polypeptide has a length of at the most about 1750 amino acids.

In a preferred embodiment, the enzyme having lactase activity is obtained from *Bifidobacterium,* preferably from *Bifidobacterium samirii, Bifidobacterium aerophilum or Bifidobacterium mongoliense,* or from *Bacillus,* preferably from *Bacillus sp. S3,* from *Varibaculum sp.,* from *Urmitella,* preferably from *Urmitella timonensis,* from *Streptococcus,* preferably from *Streptococcus entericus DSM 14446,* from *Clostridium,* preferably from *Clostridium nexile CAG:348,* or from *Neobacillus,* preferably from *Neobacillus bataviensis, Neobacillus mesonae or Neobacillus niacini,* or is a variant of an enzyme having lactase activity obtained from any of these genera or species, and has been C-terminally truncated and has a length of at the most about 1500 amino acids, such as at the most about 1400 amino acids or at the most about 1350 amino acids, for example 850-1500 amino acids, preferably 850-1400 amino acids, such as 850-1350 amino acids, more preferably 880-1350, 880-1330, 880-1320 or 885-1310 amino acids.

In the case of a lactase enzyme obtained from *Streptomyces cirratus,* it may have a length of up to about 1750 amino acids, such as up to about 1700 amino acids, e.g. up to about 1680 amino acids.

In one embodiment, the enzyme having lactase activity is obtained from *Bifidobacterium samirii* or is a variant of an enzyme having lactase activity obtained from *Bifidobacterium samirii,* and has a length of 850-1500 amino acids, preferably 1250-1400 amino acids, more preferably 1250-1350 or 1280-1320 amino acids, such as 1290-1310 amino acids. The enzyme may e.g. be derived from the polypeptide of SEQ ID NO: 1 and have a length of 850-1500 amino acids, preferably 1250-1400 amino acids, more preferably 1250-1350 or 1280-1320 amino acids, such as 1290-1310 amino acids.

In one embodiment, the enzyme having lactase activity is obtained from *Streptococcus entericus DSM 14446* or is a variant of an enzyme having lactase activity obtained from *Streptococcus entericus DSM 14446,* and has a length of 850-1500 amino acids, preferably 1250-1400 amino acids, more preferably 1250-1350 or 1270-1310 amino acids, such as 1280-1300 amino acids. The enzyme may e.g. be derived from the polypeptide of SEQ ID NO: 2 and have a length of 850-1500 amino acids, preferably 1250-1400 amino acids, more preferably 1250-1350 or 1270-1310 amino acids, such as 1280-1300 amino acids.

In one embodiment, the enzyme having lactase activity is obtained from *Varibaculum sp.* or is a variant of an enzyme having lactase activity obtained from *Varibaculum sp.,* and has a length of 850-1500 amino acids, preferably 1250-1400 amino acids, more preferably 1250-1350 or 1290-1330 amino acids, such as 1300-1320 amino acids. The enzyme may e.g. be derived from the polypeptide of SEQ ID NO: 3 and have a length of 850-1500 amino acids, preferably 1250-1400 amino acids, more preferably 1250-1350 or 1290-1330 amino acids, such as 1300-1320 amino acids.

In one embodiment, the enzyme having lactase activity is obtained from *Urmitella timonensis* or is a variant of an enzyme having lactase activity obtained from *Urmitella timonensis,* and has a length of 850-1500 amino acids, preferably 1250-1400 amino acids, more preferably 1250-1350 or 1290-1330 amino acids, such as 1300-1320 amino acids. The enzyme may e.g. be derived from the polypeptide of SEQ ID NO: 4 and have a length of 850-1500 amino acids, preferably 1250-1400 amino acids, more preferably 1250-1350 or 1290-1330 amino acids, such as 1300-1320 amino acids.

In one embodiment, the enzyme having lactase activity is obtained from *Bacillus sp.* S3 or is a variant of an enzyme having lactase activity obtained from *Bacillus sp. S3,* and has a length of 850-1500 amino acids, preferably 1250-1400 amino acids, more preferably 1250-1350 or 1290-1330 amino acids, such as 1300-1320 amino acids. The enzyme may e.g. be derived from the polypeptide of SEQ ID NO: 6 and have a length of 850-1500 amino acids, preferably 1250-1400 amino acids, more preferably 1250-1350 or 1290-1330 amino acids, such as 1300-1320 amino acids.

In one embodiment, the enzyme having lactase activity is obtained from *Bifidobacterium aerophilum* or is a variant of an enzyme having lactase activity obtained from *Bifidobacterium aerophilum,* and has a length of 850-1500 amino acids, preferably 1250-1400 amino acids, more preferably 1250-1350 or 1280-1320 amino acids, such as 1290-1310 amino acids. The enzyme may e.g. be derived from the polypeptide of SEQ ID NO: 7 and have a length of 850-1500 amino acids, preferably 1250-1400 amino acids, more preferably 1250-1350 or 1280-1320 amino acids, such as 1290-1310 amino acids.

In one embodiment, the enzyme having lactase activity is obtained from *Bifidobacterium mongoliense* or is a variant of an enzyme having lactase activity obtained from *Bifidobacterium mongoliense,* and has a length of 850-1500 amino acids, preferably 1250-1400 amino acids, more preferably 1250-1350 or 1280-1320 amino acids, such as 1290-1310 amino acids. The enzyme may e.g. be derived from the polypeptide of SEQ ID NO: 8 and have a length of 850-1500 amino acids, preferably 1250-1400 amino acids, more preferably 1250-1350 or 1280-1320 amino acids, such as 1290-1310 amino acids.

In one embodiment, the enzyme having lactase activity is obtained from *Clostridium nexile CAG:348* or is a variant of an enzyme having lactase activity obtained from *Clostridium nexile CAG:348,* and has a length of 850-1500 amino acids, preferably 1250-1400 amino acids, more preferably 1250-1350 or 1270-1310 amino acids, such as 1280-1300 amino acids. The enzyme may e.g. be derived from the polypeptide of SEQ ID NO: 9 and have a length of 850-1500 amino acids, preferably 1250-1400 amino acids, more preferably 1250-1350 or 1270-1310 amino acids, such as 1280-1300 amino acids.

In one embodiment, the enzyme having lactase activity is obtained from *Neobacillus bataviensis* or is a variant of an enzyme having lactase activity obtained from *Neobacillus bataviensis,* and has a length of 800-1400 amino acids, preferably 1000-1200 amino acids, more preferably 1100-1200 or 1110-1150 amino acids, such as 1120-1140 amino acids. The enzyme may e.g. be derived from the polypeptide of SEQ ID NO: 10 and have a length of 800-1400 amino acids, preferably 1000-1200 amino acids, more preferably 1100-1200 or 1110-1150 amino acids, such as 1120-1140 amino acids.

In one embodiment, the enzyme having lactase activity is obtained from *Neobacillus mesonae* or is a variant of an enzyme having lactase activity obtained from *Neobacillus mesonae,* and has a length of 850-1500 amino acids, preferably 1250-1400 amino acids, more preferably 1250-1350 or 1290-1330 amino acids, such as 1300-1320 amino acids. The enzyme may e.g. be derived from the polypeptide of SEQ ID NO: 11 and have a length of 850-1500 amino acids, preferably 1250-1400 amino acids, more preferably 1250-1350 or 1290-1330 amino acids, such as 1300-1320 amino acids.

In one embodiment, the enzyme having lactase activity is obtained from *Neobacillus niacini* or is a variant of an enzyme having lactase activity obtained from *Neobacillus niacini,* and has a length of 850-1500 amino acids, preferably 1250-1400 amino acids, more preferably 1250-1350 or 1290-1330 amino acids, such as 1300-1320 amino acids. The enzyme may e.g. be derived from the polypeptide of SEQ ID NO: 12 and have a length of 850-1500 amino acids, preferably 1250-1400 amino acids, more preferably 1250-1350 or 1290-1330 amino acids, such as 1300-1320 amino acids.

In one embodiment, the enzyme having lactase activity is obtained from *Streptomyces cirratus* or is a variant of an enzyme having lactase activity obtained from *Streptomyces cirratus,* and has a length of 1200-1900 amino acids, preferably 1600-1800 amino acids, more preferably 1650-1750 or 1660-1700 amino acids, such as 1670-1690 amino acids. The enzyme may e.g. be derived from the polypeptide of SEQ ID NO: 13 and have a length of 1200-1900 amino acids, preferably 1600-1800 amino acids, more preferably 1650-1750 or 1660-1700 amino acids, such as 1670-1690 amino acids.

The invention further provides a method of producing a lactose-reduced, heat-treated, milk-based product, the method comprising:

a) adding an enzyme having lactase activity to a milk-based substrate comprising at least 2% lactose (w/w), wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13,

b) after addition of the enzyme, performing a heat treatment of the milk-based substrate by holding said milk-based substrate at a holding temperature of at least 120°C for a holding time of at least 1 second followed by cooling to produce a heat-treated milk-based product, and

c) storing the heat-treated milk-based product for at least about 24 hours, preferably at least 2 days, such as at least 3 days, preferably at least 4 days at a temperature of at most 40°C,

wherein after step c) the lactose content in the milk-based product is at most 0.2% (w/w).

Preferably, after step b) but before step c), the lactose content in the milk-based product is at least 0.5% (w/w), preferably at least 1% (w/w).

The enzyme having lactase activity used in the method may, for example, comprise an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13, or to a mature polypeptide of any of SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In a preferred embodiment, the enzyme having lactase activity used in the method comprises an amino acid sequence having at least 70% sequence identity, preferably at least 75% sequence identity, such as at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4, or to a mature polypeptide of any of SEQ ID NO: 1, SEQ ID NO: 2, SEQ ID NO: 3 or SEQ ID NO: 4.

It will be apparent that the lactase enzyme used in the method of the invention may be selected from any of the lactase enzymes or variants described in more detail above and elsewhere herein.

The milk-based substrate preferably comprises 2-30%, preferably 2-17% (w/w), more preferably 4-5.5 (w/w), lactose.

The milk-based substrate may be any raw and/or processed milk material. Useful milk-based substrates include, but are not limited to, solutions/suspensions of any milk or milk-like products comprising lactose, such as whole or low-fat milk, skim milk, buttermilk, reconstituted milk powder, condensed milk, solutions of dried milk, milk comprising skim milk powder, milk permeate, whey, whey permeate, acid whey, cream, or flavored milk such as chocolate milk.

The milk-based substrate may be milk, such as raw milk, e.g., raw milk which has not been pasteurized before step a).

In a preferred embodiment, the milk-based substrate is milk, condensed milk or milk comprising skim milk powder.

In a more preferred embodiment, the milk-based substrate is milk comprising 4-5.5%, preferably 4.5-5%, lactose (w/w).

In one embodiment, the milk-based substrate is raw milk, preferably raw milk which has not been pasteurized before step a).

In one embodiment, after step a) but before step b), the milk-based substrate is incubated for at most 4 hours, preferably at most 60 minutes, more preferably at most 10 minutes, even more preferably at most 5 minutes. Such incubation may be performed at a temperature of at most 10°C, preferably at most 7°C.

However, preferably, step b) is performed immediately after step a) without a dedicated incubation step between step a) and step b).

Preferably, the time from addition of the enzyme until the holding temperature of step b) is reached is at most 5 minutes, more preferably at most 2 minutes, even more preferably at most 1 minute.

In a preferred embodiment, the heat treatment is performed as an indirect heat treatment, preferably an indirect UHT treatment. Pumping equipment may have been fitted to add the enzyme to the milk-based substrate while said milk-based substrate is streaming through process equipment, such as process pipes. A heating medium may be used, such as a plate heat-exchanger or a tubular heat-exchanger, which is not in direct contact with the milk-based substrate but separated by equipment contact surfaces. Preferably, the enzyme is added to the milk-based substrate immediately before the heat treatment step, such as immediately before the holding temperature is reached, optionally while the temperature of the milk-based substrate is increasing towards the holding temperature of step b).

In another preferred embodiment, the heat treatment is performed as a direct heat treatment, preferably a direct UHT treatment. The heat treatment may be performed by steam injection or steam infusion, preferably steam injection, using high-pressure steam to heat the milk-based substrate. In a preferred embodiment, the enzyme is added with the steam. Preferably, after the holding time of step b), the milk-based substrate comprising the steam is flash-cooled in a vacuum to remove water equivalent to amount of condensed steam used.

A combination of direct and indirect heat treatment may also be used.

The heat treatment may be an ESL treatment, an ultra-pasteurization or a UHT treatment.

The heat treatment may be performed at a temperature of 120-150°C.

In one embodiment, the heat treatment is performed at a temperature of at least 123°C, preferably at 123-145°C.

In one embodiment, the heat treatment is performed at a temperature of at least 130°C, preferably at 130-145°C.

In one embodiment, the heat treatment is performed at a temperature of at least 138°C, preferably at 138-145°C, more preferably at 138-142°C.

The holding time of step b) may be 1-30 seconds, preferably 1-10 seconds, more preferably 1-5 seconds.

In one embodiment, the heat treatment is a UHT treatment, preferably a UHT treatment performed at a temperature of 130-145°C for a time of 1-30 seconds, more preferably at a temperature of 138-145°C for a time of 1-10 seconds, even more preferably at a temperature of 138-144°C for a time of 1-5 seconds.

In one embodiment, the heat treatment is a UHT treatment performed at a temperature of 128-132°C for 25-35 seconds, at a temperature of 138-140°C for 2-5 seconds or at a temperature of 144-146°C for 1-2 seconds.

In one embodiment, the heat treatment is an ESL treatment or an ultra-pasteurization, preferably an ESL treatment or an ultra-pasteurization performed at a temperature of 120-140°C for a time of 1-5 seconds, more preferably an ESL treatment or an ultra-pasteurization performed at a temperature of 120-130°C for 1-5 seconds or at a temperature of 138-140°C for 2-4 seconds.

Preferably, no enzyme having lactase activity is added to the milk-based product after step b), such as after the holding time of step b). More preferably, no enzyme is added to the milk-based product after step b), such as after the holding time of step b). Even more preferably, nothing is added to the milk-based product after step b), such as after the holding time of step b). This is because, after the heat-treatment, the milk-based product is sterile, and adding anything, even something which is considered sterile, will be at the risk of contaminating the product.

After the holding time of step b), the milk-based product is cooled to at most 40°C, preferably at most 35°C, more preferably at most 30°C, preferably within 5 minutes, more preferably within 3 minutes, even more preferably within 2 minutes, such as within 1 minute.

Preferably, the time from addition of the enzyme until the heat-treated milk-based product has been cooled to a temperature of at most 40°C, preferably at most 35°C, more preferably at most 30°C, is at most 3.5 minutes, preferably at most 3 minutes, more preferably at most 2.5 minutes, such as at most 2 minutes or at most 1 minute.

After step b) but before step c) the milk-based product may be homogenized.

Alternatively, homogenization may be performed before reaching the holding temperature of step b). In indirect UHT treatments (e.g., tube exchange or plate exchange), homogenization is preferably performed upstream. In direct UHT treatments (e.g., steam injection or steam infusion), homogenization is preferably performed downstream.

Preferably, after step b) but before step c) the milk-based product is aseptically packed.

In a preferred embodiment, the milk-based product is UHT milk. UHT milk in the context of the present invention is milk which has been subjected to a sterilization procedure which is intended to kill all microorganisms, including the bacterial spores.

Preferably, less than 80% of the lactose has been hydrolyzed when step b) is completed, and more than 90% of the lactose has been hydrolyzed after one week. More preferably, less than 60% of the lactose has been hydrolyzed when step b) is completed, and more than 95% of the lactose has been hydrolyzed after one week.

Preferably, after step b), the enzyme retains at least 0.1% of its initial activity, more preferably at least 0.5%, more preferably at least 1%, more preferably at least 2%, more preferably at least 10%, more preferably at least 50%, even more preferably at least 80%, most preferably at least 90%. The enzyme activity retained after step b) in percent of the enzyme activity when the enzyme is added may be determined, e.g., by using a "Residual activity assay" of the examples.

Preferably, step c) is performed at a temperature of 2-40°C, preferably 15-40°C, more preferably 18-40°C, most preferably 18-30°C. In a preferred embodiment, step c) is performed at room temperature which may vary during the storage period.

In a preferred embodiment, after step b) but before step c) the lactose content in the milk-based product is at least 1% (w/w) , such as at least 2% (w/w), at least 3% (w/w) or at least 4% (w/w).

In another preferred embodiment, after step b) but before step c) the lactose content in the milk-based product has been reduced by at most 80%, preferably at most 50%, more preferably at most 20%, compared to the lactose content before step a).

In step c), the heat-treated milk-based product is stored for at least about 24 hours, preferably at least 2 days, such as at least 3 days, preferably at least 4 days, preferably at least 7 days, more preferably at least 14 days, such as at least 21 days. It is primarily during this storage period that the lactose level is decreased to the desired level by the residual activity of the lactase.

In a preferred embodiment, after having stored the heat-treated milk-based product for 21 days at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

In another preferred embodiment, after having stored the heat-treated milk-based product for 14 days at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

In another preferred embodiment, after having stored the heat-treated milk-based product for 7 days at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

In another preferred embodiment, after having stored the heat-treated milk-based product for 4 days at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

In another preferred embodiment, after having stored the heat-treated milk-based product for 3 days at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

In a preferred embodiment, after having stored the heat-treated milk-based product for 21 days at a temperature of 2-40°C, preferably 15-40°C, more preferably 18-40°C, most preferably 18-30°C, the lactose content in the milk-based product has been reduced by at least 80% or at least 85%, preferably at least 90, 91, 92, 93, 94, 95, 96 or 97%, more preferably at least 98%, even more preferably at least 99% or at least 99.5%, and most preferably at least 99.8% or at least 99.9%, compared to the lactose content before step a).

In another preferred embodiment, after having stored the heat-treated milk-based product for 14 days at a temperature of 2-40°C, preferably 15-40°C, more preferably 18-40°C, most preferably 18-30°C, the lactose content in the milk-based product has been reduced by at least 80% or at least 85%, preferably at least 90, 91, 92, 93, 94, 95, 96 or 97%, more preferably at least 98%, even more preferably at least 99% or at least 99.5%, and most preferably at least 99.8% or at least 99.9%, compared to the lactose content before step a).

In another preferred embodiment, after having stored the heat-treated milk-based product for 7 days at a temperature of 2-40°C, preferably 15-40°C, more preferably 18-40°C, most preferably 18-30°C, the lactose content in the milk-based product has been reduced by at least 80% or at least 85%, preferably at least 90, 91, 92, 93, 94, 95, 96 or 97%, more preferably at least 98%, even more preferably at least 99% or at least 99.5%, and most preferably at least 99.8% or at least 99.9%, compared to the lactose content before step a).

In another preferred embodiment, after having stored the heat-treated milk-based product for 4 days at a temperature of 2-40°C, preferably 15-40°C, more preferably 18-40°C, most preferably 18-30°C, the lactose content in the milk-based product has been reduced by at least 80% or at least 85%, preferably at least 90, 91, 92, 93, 94, 95, 96 or 97%, more preferably at least 98%, even more preferably at least 99% or at least 99.5%, and most preferably at least 99.8% or at least 99.9%, compared to the lactose content before step a).

In another preferred embodiment, after having stored the heat-treated milk-based product for 3 days at a temperature of 2-40°C, preferably 15-40°C, more preferably 18-40°C, most preferably 18-30°C, the lactose content in the milk-based product has been reduced by at least 80% or at least 85%, preferably at least 90, 91, 92, 93, 94, 95, 96 or 97%, more preferably at least 98%, even more preferably at least 99% or at least 99.5%, and most preferably at least 99.8% or at least 99.9%, compared to the lactose content before step a).

In a preferred embodiment, after having stored the heat-treated milk-based product for 21 days at a temperature of 2-40°C, preferably 15-40°C, more preferably 18-40°C, most preferably 18-30°C, the lactose content in the milk-based product is at most 1000 ppm, preferably at most 100 ppm.

In another preferred embodiment, after having stored the heat-treated milk-based product for 14 days at a temperature of 2-40°C, preferably 15-40°C, more preferably 18-40°C, most preferably 18-30°C, the lactose content in the milk-based product is at most 1000 ppm, preferably at most 100 ppm.

In another preferred embodiment, after having stored the heat-treated milk-based product for 7 days at a temperature of 2-40°C, preferably 15-40°C, more preferably 18-40°C, most preferably 18-30°C, the lactose content in the milk-based product is at most 1000 ppm, preferably at most 100 ppm.

In another preferred embodiment, after having stored the heat-treated milk-based product for 4 days at a temperature of 2-40°C, preferably 15-40°C, more preferably 18-40°C, most preferably 18-30°C, the lactose content in the milk-based product is at most 1000 ppm, preferably at most 100 ppm.

In another preferred embodiment, after having stored the heat-treated milk-based product for 3 days at a temperature of 2-40°C, preferably 15-40°C, more preferably 18-40°C, most preferably 18-30°C, the lactose content in the milk-based product is at most 1000 ppm, preferably at most 100 ppm.

After step c), the lactose-reduced heat-treated milk product may, if desired, be freeze-dried.

The enzyme having lactase activity may be added at a concentration of 100-50,000, preferably 500-40,000 LAU(B) per liter milk-based substrate.

The enzyme having lactase activity may be added at a concentration of 1-150 mg enzyme protein per liter milk-based substrate, preferably 1-100, more preferably 2-50 or 5-50 mg enzyme protein per liter milk-based substrate.

Before step b), a reducing agent may be added to the milk-based substrate, preferably a food-approved reducing agent, more preferably a reducing agent selected among L-cysteine, sulphite and glutathione. The reducing agent may be added together with the enzyme, for example the reducing agent may be part of the enzyme formulation. The reducing agent may be added to lower or prevent oxidation of, e.g., cysteines in the lactase enzyme which do not form part of a disulfide bridge, sometimes referred to as "free cysteines". Oxidation of such free cysteines may lower the ability of the enzyme to refold after the thermal treatment, such as the UHT treatment.

The lactase enzyme of SEQ ID NO: 5 has a free cysteine residue at position C372, the oxidation of which has been shown to lower the ability of the enzyme to refold, and a corresponding cysteine residue can be identified in many other lactase enzymes by aligning their amino acid sequence with SEQ ID NO: 5. In a preferred embodiment, the enzyme having lactase activity has an amino acid substitution of the cysteine corresponding to C372 of SEQ ID NO: 5, preferably to serine, alanine or glycine.

Preferably, the enzyme having lactase activity is a lactase. Preferably, the enzyme belongs to enzyme class 3.2.1.21, 3.2.1.23 or 3.2.1.108, more preferably 3.2.1.23 or 3.2.1.108.

In a preferred embodiment, the enzyme having lactase activity is a neutral lactase.

In a preferred embodiment, the enzyme having lactase activity is purified.

In a preferred embodiment, the enzyme having lactase activity is isolated.

Preferably, the enzyme having lactase activity has a temperature optimum of 30-60°C, preferably 35-55°C. The temperature optimum may be determined using Method 2 of the examples.

Preferably, the enzyme having lactase activity has a melting temperature Tm of 50-70°C determined by thermal shift at pH 6. In another preferred embodiment, the enzyme having lactase activity has a melting temperature Tm of 50-70°C determined by thermal shift at pH 7. The melting temperature Tm may be determined using the thermal shift assay of Example 2.

Preferably, the enzyme having lactase activity has a residual activity of at least 1%, preferably at least 2%, more preferably at least 5%, even more preferably at least 10%, after incubation at 70°C for 30 seconds and 140°C for 5 seconds in skimmed milk having a lactose content of 4.7%. The residual activity may be determined as described in the examples.

In a preferred embodiment, the enzyme having lactase activity has an initial lactose turn-over of at least 10 per second per enzyme molecule in milk at 5°C. The initial lactose turn-over may be determined, e.g., when 0-10% of the lactose has been hydrolyzed. The skilled person will know how to determine the initial lactose turn-over per enzyme molecule. For example, it can be a direct measurement of lactose using HPLC or indirect using a glucose detection method.

The initial lactose turn-over per enzyme molecule in milk at 5°C is about 36 $sec^{-1}$ for Lactozym® Pure and about 89 $sec^{-1}$ for Saphera®, both available from Novozymes A/S, Denmark.

In a more preferred embodiment, the enzyme having lactase activity has an initial lactose turn-over of at least 20, preferably at least 50, more preferably at least 80, per second per enzyme molecule in milk at 5°C.

In a preferred embodiment, the enzyme having lactase activity has an average lactose turn-over from initial lactose, preferably about 4.7% lactose, to 0.1% residual lactose of at least 10 per second per enzyme molecule in milk at 5°C.

The average lactose turn-over from initial lactose of 4.7% to 0.1% residual lactose is about 9 $sec^{-1}$ for Lactozym® Pure and about 23 $sec^{-1}$ for Saphera®. The reason for lower average lactose turnover for 4.7% to 0.1% compared to initial rate (4.7% to 4.23%) is a combination of several factors, e.g. higher product inhibition and lower substrate concentration close to 0.1%.

In a preferred embodiment, the enzyme having lactase activity has an average lactose turn-over from initial lactose, preferably about 4.7%, to 0.01% residual lactose of at least 5 per second per enzyme molecule in milk at 5°C.

The average lactose turn-over from initial lactose of 4.7% to 0.01% residual lactose is about 5 $sec^{-1}$ for Lactozym® Pure and about 18 $sec^{-1}$ for Saphera®.

In a preferred embodiment, the enzyme having lactase activity has a Michaelis constant $K_M$ of at most 40 mM, preferably at most 30 mM, more preferably at most 20 mM, at 5°C.

In another preferred embodiment, the enzyme having lactase activity has a Michaelis constant $K_M$ of at most 40 mM, preferably at most 30 mM, more preferably at most 20 mM, at 37°C.

The Michaelis constant $K_M$ is the substrate concentration, in this case the lactose concentration, at which the reaction rate is at half-maximum and is a measure of the substrate's affinity for the enzyme. A small $K_M$ indicates high affinity, meaning that the rate will approach its maximum with lower substrate concentration than with a larger $K_M$.

The Michaelis constant $K_M$ may be determined according to the method of Example 5 of WO09071539A1. In this example, $K_M$ at 5°C is determined as 8 mM for the experimental *Bifidobacterium* lactase and 30 mM for Lactozym (K. *lactis* lactase), and $K_m$ at 37°C is determined as 13 mM for the experimental *Bifidobacterium* lactase and 30 mM for Lactozym.

**PREFERRED EMBODIMENTS**

1. A milk-based product comprising an enzyme having lactase activity, wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

2. The milk-based product of embodiment 1, wherein the enzyme having lactase activity comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

3. The milk-based product of embodiment 2, wherein the enzyme having lactase activity comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4.

4. The milk-based product of embodiment 3, wherein the enzyme having lactase activity is selected from the group consisting of:

a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;

b) a variant of SEQ ID NO: 2 comprising at least one substitution selected from the group consisting of P52A, G607T, L1064C and Y1110C, for example two or more of said substitutions, wherein numbering is based on SEQ ID NO: 2; and

c) a variant of SEQ ID NO: 4 comprising the substitution G386Q and/or P620T, wherein numbering is based on SEQ ID NO: 4.

5. The milk-based product of any of embodiments 1-4, wherein the enzyme having lactase activity has a length of at the most about 1500 amino acids, such as at the most about 1400 amino acids or at the most about 1350 amino acids, for example 850-1500 amino acids, preferably 850-1400 amino acids, such as 850-1350 amino acids.

6. The milk-based product of any of embodiments 1-5, wherein the product has been heat-treated and has a lactose content of at most 0.2% (w/w).

7. The milk-based product of any of embodiments 1-6, wherein the product is UHT milk, ESL milk or ultra-pasteurized milk.

8. An enzyme having lactase activity, wherein the enzyme is selected from the group consisting of:

a) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 1, wherein the polypeptide has a length of at the most about 1500 amino acids, such as at the most about 1400 amino acids or at the most about 1350 amino acids;

b) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 2, wherein the polypeptide has a length of at the most about 1500 amino acids, such as at the most about 1400 amino acids or at the most about 1350 amino acids;

c) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 3, wherein the polypeptide has a length of at the most about 1500 amino acids, such as at the most about 1400 amino acids or at the most about 1350 amino acids;

d) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 4, wherein the polypeptide has a length of at the most about 1500 amino acids, such as at the most about 1400 amino acids or at the most about 1350 amino acids;

e) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 6, wherein the polypeptide has a length of at the most about 1500 amino acids, such as at the most about 1400 amino acids or at the most about 1350 amino acids;

f) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 7, wherein the polypeptide has a length of at the most about 1500 amino acids, such as at the most about 1400 amino acids or at the most about 1350 amino acids;

g) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 8, wherein the polypeptide has a length of at the most about 1500 amino acids, such as at the most about 1400 amino acids or at the most about 1350 amino acids;

h) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 9, wherein the polypeptide has a length of at the most about 1500 amino acids, such as at the most about 1400 amino acids or at the most about 1350 amino acids;

i) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 10, wherein the polypeptide has a length of at the most about 1500 amino acids, such as at the most about 1400 amino acids or at the most about 1350 amino acids;

j) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 11, wherein the polypeptide has a length of at the most about 1500 amino acids, such as at the most about 1400 amino acids or at the most about 1350 amino acids;

k) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 12, wherein the polypeptide has a length of at the most about 1500 amino acids, such as at the most about 1400 amino acids or at the most about 1350 amino acids; and

l) a polypeptide having an amino acid sequence which is at least 60%, such as at least 70%, at least 75%, at least 80%, at least 85%, at least 90%, at least 95%, at least 98%, at least 99%, at least 99.5% or 100% identical to SEQ ID NO: 13, wherein the polypeptide has a length of at the most about 1750 amino acids, such as at the most about 1700 amino acids.

9. The enzyme of embodiment 8, wherein the enzyme has lactase activity and is selected from the group consisting of:

a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;

b) a variant of SEQ ID NO: 2 comprising at least one substitution selected from the group consisting of P52A, G607T, L1064C and Y1110C, for example two or more of said substitutions, wherein numbering is based on SEQ ID NO: 2; and

c) a variant of SEQ ID NO: 4 comprising the substitution G386Q and/or P620T, wherein numbering is based on SEQ ID NO: 4.

10. A method of producing a lactose-reduced, heat-treated, milk-based product, the method comprising:

a) adding an enzyme having lactase activity to a milk-based substrate comprising at least 2% lactose (w/w), wherein the enzyme having lactase activity comprises an amino acid sequence having at least 60% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13,

b) after addition of the enzyme, performing a heat treatment of the milk-based substrate by holding said milk-based substrate at a holding temperature of at least 120°C for a holding time of at least 1 second followed by cooling to produce a heat-treated milk-based product, and

c) storing the heat-treated milk-based product for at least about 24 hours, preferably at least 2 days, such as at least 3 days, preferably at least 4 days at a temperature of at most 40°C,

wherein after step c) the lactose content in the milk-based product is at most 0.2% (w/w), and preferably wherein, after step b) but before step c), the lactose content in the milk-based product is at least 0.5% (w/w), preferably at least 1% (w/w).

11. The method of embodiment 10, wherein the enzyme having lactase activity comprises an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4.

12. The method of embodiment 11, wherein the enzyme having lactase activity is selected from the group consisting of:

    a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;

    b) a variant of SEQ ID NO: 2 comprising at least one substitution selected from the group consisting of P52A, G607T, L1064C and Y1110C, for example two or more of said substitutions, wherein numbering is based on SEQ ID NO: 2; and

    c) a variant of SEQ ID NO: 4 comprising the substitution G386Q and/or P620T, wherein numbering is based on SEQ ID NO: 4.

13. The method of any of embodiments 10-12, wherein step b) is performed immediately after step a) without a dedicated incubation step between step a) and step b).

14. The method of any of embodiments 10-13, wherein pumping equipment has been fitted to add the enzyme to the milk-based substrate while said milk-based substrate is streaming through process equipment, such as process pipes.

15. The method of any of embodiments 10-14, wherein the time from addition of the enzyme until the holding temperature of step b) is reached is at most 5 minutes, preferably at most 2 minutes, more preferably at most 1 minute.

16. The method of any of embodiments 10-15, wherein the time from addition of the enzyme until the heat-treated milk-based product has been cooled to a temperature of at most 40°C, preferably at most 35°C, more preferably at most 30°C, is at most 3.5 minutes, preferably at most 3 minutes, more preferably at most 2.5 minutes, such as at most 2 minutes or at most 1 minute.

17. The method of any of embodiments 10-16, wherein the milk-based substrate comprises 2-30%, preferably 2-17%, more preferably 4-5.5%, lactose (w/w).

18. The method of any of embodiments 10-17, wherein the milk-based substrate is milk comprising 4-5.5% lactose (w/w).

19. The method of any of embodiments 10-18, wherein the heat treatment is an ESL treatment, an ultra-pasteurization or a UHT treatment, preferably a UHT treatment.

20. The method of any of embodiments 10-19, wherein the heat treatment is a UHT treatment performed at a temperature of 128-132°C for 25-35 seconds, at a temperature of 138-140°C for 2-5 seconds or at a temperature of 144-146°C for 1-2 seconds.

21. The method of any of embodiments 10-20, where after the holding time of step b), the milk-based substrate is cooled to at most 40°C, preferably at most 35°C, more preferably at most 30°C, preferably within 5 minutes, more preferably within 3 minutes, even more preferably within 2 minutes, such as within 1 minute.

22. The method of any of embodiments 10-21, wherein after step b) but before step c) the milk-based product is aseptically packed.

23. The method of any of embodiments 10-22, wherein after step b), the enzyme retains at least 0.1% of its initial activity, preferably at least 0.5%, more preferably at least 1%, more preferably at least 2%, more preferably at least 10%, more preferably at least 50%, even more preferably at least 80%, most preferably at least 90%.

24. The method of any of embodiments 10-23, wherein after having stored the heat-treated milk-based product for 21 days, preferably 14 days, more preferably 7 days, even more preferably 4 days, most preferably 3 days, at a temperature of 2-40°C, preferably 15-40°C, more preferably 18-40°C, most preferably 18-30°C, the lactose content in the milk-based product is at most 0.2% (w/w), preferably at most 0.1%, more preferably at most 0.01%.

25. The method of any of embodiments 10-24, wherein the enzyme having lactase activity has a temperature optimum of 30-60°C, preferably 35-55°C.

26. The method of any of embodiments 10-25, wherein the enzyme having lactase activity has a residual activity of at least 1%, preferably at least 2%, more preferably at least 5%, even more preferably at least 10%, after incubation at 70°C for 30 seconds and 140°C for 5 seconds in skimmed milk having a lactose content of 4.7%.

27. The method of any of embodiments 10-26, wherein the enzyme having lactase activity has a residual activity of at least 0.1%, preferably at least 0.5%, at least 1% or at least 2%, more preferably at least 5%, even more preferably at least 10%, after incubation in skimmed milk having a lactose content of 4.7% at 90°C for 30 seconds, at 140°C for 5 seconds and at 70°C for 30 seconds followed by cooling to 0-10°C and subsequent incubation at 23°C for 0.5 hours, wherein the residual activity is relative to the activity of the same enzyme in skimmed milk without incubation at 90°C for 30 seconds, at 140°C for 5 seconds and at 70°C for 30 seconds followed by cooling to 0-10°C and subsequent incubation at 23°C for 0.5 hours.

28. The method of any of embodiments 10-27, wherein the enzyme has a residual activity of at least 0.5%, preferably at least 1%, at least 2% or at least 3%, more preferably at least 5%, even more preferably at least 10%, after incubation in skimmed milk having a lactose content of 4.7% at 90°C for 30 seconds, at 140°C for 5 seconds and at 70°C for 30 seconds followed by cooling to 0-10°C and subsequent incubation at 23°C for 72 hours, wherein the residual activity is relative to the activity of the same enzyme in skimmed milk without incubation at 90°C for 30 seconds, at 140°C for 5 seconds and at 70°C for 30 seconds followed by cooling to 0-10°C and subsequent incubation at 23°C for 72 hours.

29. The method of any of embodiments 10-28, wherein the enzyme having lactase activity is added at a concentration of 1-150 mg enzyme protein per liter milk-based substrate, preferably 1-100, more preferably 2-50 or 5-50 mg enzyme protein per liter milk-based substrate.

30. The milk-based product of any of embodiments 1-7, or the method of any of embodiments 10-29, wherein the enzyme is as defined in embodiment 8.

31. A method of producing a lactose-reduced milk product, the method comprising adding an enzyme having lactase activity to a milk-based substrate comprising at least 2% lactose (w/w), and subjecting the milk-based substrate comprising the enzyme to a heat treatment at a temperature of at least 120°C for a holding time of at least 1 second, wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

32. Use of an enzyme having lactase activity for producing a lactose-reduced, heat-treated, milk-based product in a method wherein a milk-based substrate comprising at least 2% lactose (w/w) to which the enzyme has been added is subjected to a heat treatment at a temperature of at least 120°C for a holding time of at least 1 second, wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

The invention is further described in the following non-limiting examples.

**EXAMPLES**

**Assay methods**

**Method 1:**

*LAU(B) assay*

The activity in LAU-B/g of a specific lactase may be determined by direct measurement of o-nitrophenyl (ONP) released from o-nitrophenyl β-D-galactopyranoside (ONPG) in a buffer containing 1.46 mg/ml substrate in 0.05 M MES, 1 mM MgSO4 7H2O, 450 mg/L Brij™ 35 at pH 6.5 and 30°C. After 600 seconds incubation, the reaction is stopped by adding 0.2 M Na2CO3 and the released ONP is measured at 405 nm after 126 seconds incubation. The activity is obtained by comparing to a standard curve run with a lactase of known activity, and the activity of the unknown sample calculated from this. The lactase of known activity may, e.g., be Saphera®, available from Novozymes A/S, Denmark.

**Method 2:**

*Assay for determining temperature optimum*

A temperature profile to determine the temperature optimum is prepared by adding 10 μl diluted enzyme samples (diluted with 50 mM succinate, 50 mM HEPES, 50 mM CHES, 150 mM KCl, 2 mM CaCl2, 1 mM MgCl2 + 0.01% Triton X-100, pH 6.5) to PCR tubes. Then 90 μl substrate (167 mM lactose, 50 mM succinate, 50 mM HEPES, 50 mM CHES, 150 mM KCl, 2 mM CaCl2, 1 mM MgCl2, pH 6.5) is added and the PCR tubes are placed in a preheated PCR block with a temperature gradient of 35-75°C (using TProfessional thermocycler, Biometra) and incubated for 30 min at 35-75°C (gradient), and then placed on ice. The reaction is stopped by adding 100 μl 0.25 M NaOH. Twenty μl is transferred to a 96-well microtiter plate, and 230 μL GOD-Perid (100 mM potassium phosphate buffer, pH 7, 0.6 g/l glucose oxidase, 0.02 g/l horseradish peroxidase, 1.0 g/l ABTS) solution is added. After 30 minutes in the dark at room temperature the absorbance is measured at 420 nm. The initial dilution of the enzyme should be adjusted so the final 420 nm absorbance reading at the optimum temperature is between 0.5 - 2.5.

The temperature with the highest delta Abs at 420 nm ("Abs 420 nm with enzyme" minus "Abs 420 nm without enzyme", i.e. background) is set to 100% (temperature optimum), and relative activity at other temperatures based on delta Abs 420 nm in relation to the delta Abs 420 nm with the highest value is used to determine a temperature profile.

**Example 1**

*Testing of novel lactase enzymes and variants*

In this example, a number of newly identified lactases and variants thereof are demonstrated to be useful in the method of the present invention for the production of heat-treated milk products such as UHT milk. The lactases are added to the milk just before UHT treatment and have been shown to have sufficient residual activity after the UHT treatment to efficiently hydrolyze the lactose in the milk during a few days of storage at room temperature.

Some of the lactases are newly identified wild-type enzymes which have been C-terminally truncated to facilitate expression as secreted enzymes, while others are variants of these wild-type enzymes in which one or more amino acids have been substituted using established protein engineering techniques. The enzymes tested in this example are listed in Table 1 below.

*Expression in Bacillus subtilis*

The genes encoding the enzymes were optimized for expression in *Bacillus subtillis* using standard methods known in the art, except for the lactases of SEQ ID NO: 10 (*Neobacillus bataviensis*) and SEQ ID NO: 13 (*Streptomyces cirratus*), where the native DNA (of the truncated mature peptide) was used. The genes were fused with DNA encoding a *Bacillus clausii* secretion signal (encoding the following amino acid sequence: MKKPLGKIVASTALLISVAFSSSIASA (SEQ ID NO: 14)) replacing the native secretion signal. Furthermore, the expression construct results in the addition of an amino-terminal poly histidine tag consisting of the amino acid sequence HHHHHHPR (SEQ ID NO: 15)) to the mature lactase to facilitate easy purification by immobilized metal affinity chromatography. The resulting gene was ordered as a fully synthetically produced DNA fragment from Twist Bioscience (San Francisco, CA, USA).

The linear integration constructs were SOE-PCR fusion products (Horton, R.M., Hunt, H.D., Ho, S.N., Pullen, J.K. and Pease, L.R. (1989) Engineering hybrid genes without the use of restriction enzymes, gene splicing by overlap extension, Gene 77: 61-68) made by fusion of the gene of interest between two *B. subtilis* chromosomal regions along with strong promoters and a chloramphenicol resistance marker. The SOE-PCR method is also described in patent application WO 2003/095658.

The lactase genes were expressed under the control of a triple promoter system (as described in WO 99/43835), consisting of the promoters from *Bacillus licheniformis* alpha-amylase gene (amyL), *Bacillus amyloliquefaciens* alpha-amylase gene (amyQ), and the *Bacillus thuringiensis* cryIIIA promoter including stabilizing sequence.

For each of the lactase expression constructs, the SOE-PCR product was transformed into *Bacillus subtilis* and integrated in the chromosome by homologous recombination into the pectate lyase locus. Subsequently, a recombinant *Bacillus subtilis* clone containing the integrated expression construct was grown in liquid culture. The culture broth was centrifuged (20,000 x g, 20 min) and the supernatant was carefully decanted from the precipitate and used for purification of the enzyme or, alternatively, sterile filtered supernatant was used directly for assays.

*Purification of the recombinant enzyme by immobilized metal affinity chromatography*

The pH of the cleared supernatant was adjusted to pH 8, filtrated through a 0.2 $\mu$M filter, and the supernatant was applied to a 5 ml HisTrap™ Excel column. Prior to loading, the column had been equilibrated in 5 column volumes (CV) of 50 mM Tris/HCl pH 8. To remove unbound material, the column was washed with 8 CV of 50 mM Tris/HCl pH 8, and elution of the target was obtained with 50 mM HEPES pH 7 + 10 mM imidazole. The eluted protein was desalted on a HiPrep™ 26/10 desalting column, equilibrated using 3 CV of 50 mM HEPES pH 7 + 100 mM NaCl. This buffer was also used for elution of the target, with a flow rate of 10 ml/min. Relevant fractions were selected and pooled based on the chromatogram and SDS-PAGE analysis.

*UHT treatment*

The enzymes of Table 1 were added to skimmed milk and a UHT treatment was performed, followed by incubation at 23°C for 0.5 hrs and for 72 hrs, with measurement of lactase activity and lactose being performed after each of these two intervals as described below.

Between 5.5 and 31.6 mg enzyme protein (ep) per liter skimmed milk was used, depending on the enzyme. For most of the enzymes, 12.7 mg ep/L skimmed milk was used (see Table 2, column 2, below). Sodium azide* was added to a final concentration of 0.025% (w/v) in all milk tested to avoid microbial growth, since due to handling etc. the tubes are not fully sterile despite the UHT step. The milk samples were applied to a laboratory scale UHT setup as described below. A syringe with a 10 ml milk sample was connected to a long Teflon tube (internal diameter of 0.8 mm) that was immersed sequentially in four baths. The first bath was in silicone at 90°C with 3 m of the Teflon tube, the second connected bath was in silicone at 140°C with 50 cm of the Teflon tube, the third silicone bath was at 70°C with 3 m of Teflon tube, and the final bath was an ice/water bath (0°C) with 1 m of Teflon tube to cool the milk. A flow of 3 ml/min was applied to the syringe, ensuring that the milk samples were incubated at 90°C for 30 sec, 140°C for 5 sec and 70°C for 30 sec before cooling for 10 sec in the ice/water bath, thereby cooling the milk to a temperature in the range of 0-10°C. Finally, the milk was collected in tubes after the ice/water bath, and the samples were incubated at 23°C for 0.5 h and for 72 h, and then assayed for residual activity and lactose amount using High-Performance Anion-Exchange Chromatography with Pulsed Amperometric Detection (HPAEC-PAD).

> \* Sodium azide is not to be used in a commercial process, where sterility is ensured by other means, but is used in this lab setup which may not be completely sterile and where the milk is not to be consumed.

*Residual activity assay*

Samples were centrifuged at 20,600 g in a precooled centrifuge for 45 min at 5°C, and the supernatant was diluted with 20 mM sodium succinic acid and 0.01% Triton X-100, pH 6.5, so an absorbance reading below 1.5 at 405 nm could be measured. Twenty-five $\mu$l of each sample was mixed with 175 $\mu$l of ONPG substrate (1.67 mg/ml ONPG (o-NitroPhenyl $\beta$-D-galatopyranoside, ~5.5 mM), 0.05 M MES, 1 mM $MgSO_4$, 150 mM KCl, 0.01% Triton X-100, pH 6.5 (preferably adjusted with NaOH)) and incubated for 2.5 h at 40°C and stopped by adding 50 $\mu$l $Na_2CO_3$ + 5 mM $Na_4EDTA$ and measured at 405 nm. Residual activity in % was calculated using the formula = ((Abs405$^{heat\text{-}treated\ sample}$ - Abs405$^{blank}$) * dilution factor) / ((Abs405$^{untreated\ sample}$ - Abs405$^{blank}$) * dilution factor) * 100%.

The heat-treated sample contains enzyme mixed with skimmed milk and sodium azide and is subjected to UHT treatment and subsequent cooling as described above followed by incubation at 23°C for 0.5 h and 72 h. The untreated sample is the same enzyme mixed with skimmed milk and sodium azide but without UHT treatment and incubation. Both samples were diluted to obtain an absorption at 405 nm in the range of 0.5-1.0. The blank is sample without enzyme, where the same dilution factor has been used as for the sample with enzyme. The results are shown in Tables 2 and 3 below.

*Analysis of residual lactose content*

The analysis of residual lactose was performed by high-performance anion exchange chromatography coupled with pulsed amperometry detector (HPAEC-PAD).

*Sample preparation for HPAEC-PAD*

The enzyme was inactivated by adding 5 μl glacial acetic acid to 1 ml milk sample, after which the sample was heated to 90°C for 5 min and centrifugated at 14,200 rpm for 10 min. 50 μl sample was transferred to a 5 ml Eppendorf tube containing 500 μl MQ (Milli-Q) water. 10 μl Carrez I solution was added and mixed, and then 10 μl Carrez II solution was added and mixed. Then, 4.43 ml MQ water (total volume of 5 ml) was added and mixed. Centrifugation was carried out at 14,200 rpm for 5 min. The supernatant was diluted x5 with Milli-Q water. These samples were analyzed on HPAEC-PAD.

*Lactose determination using HPAEC-PAD*

The analysis performed is essentially as described in Leeuwen S, Kuipers B, Dijkhuizen L, Ka-merling J. Comparative structural characterization of 7 commercial galacto-oligosaccharide (GOS) products, Carbohydrate Research, 425 (2016) 48-58 with minor modifications, e.g., with a shorter gradient as specified below. A Dionex™ ICS-6000 workstation (Dionex, Amsterdam, The Netherlands) was used, equipped with a CarboPac™ PA1 4 x 50 mm Guard Column (Dionex, product no. 043096) followed by CarboPac PA1 4 x 250 mm (Dionex, product no. 035391) and an ICS-6000 DC ECD detector (Dionex), using a complex gradient of A: Milli-Q water, B: 600 mM NaOAc in 100 mM NaOH, C: 100 mM NaOH, and D: 50 mM NaOAc. The fractionations were performed at 1.0 mL/min with 85% A, 0% B, 10% C, and 5% D in 25 min linear gradient to 10% A, 0% B, 40%C, and 50% D, followed by a 2-min linear gradient to 0% A, 25% B, 75% C and 0% D, directly followed by 5 min washing with 100% B and reconditioning for 15 min with 85% A, 0% B, 10% C, and 5% D. A lactose standard was used to determine the amount of lactose in the enzyme treated samples. The results are shown in Table 2 below, columns 3 and 4.

*Dose-response curve for non-UHT treated B. bifidum lactase*

To be able to compare the activities of the enzymes in the UHT-treated samples to the activity of non-UHT treated *B. bifidum* lactase (enzyme #15, SEQ ID NO: 5), a dose/response curve for the non-UHT treated *B. bifidum* lactase (enzyme #15, SEQ ID NO: 5) was made.
The following doses of *B. bifidum* wild-type lactase (enzyme #15) in skim milk (+ 0.025% sodium azide) were made in duplicate: 2.03, 1.63, 1.30, 1.04, 0.832, 0.666, 0.532, 0.426, 0.341, 0.273, 0.218, 0.174, 0.140, 0.112, 0.0893, 0.0715 mg ep/liter skim milk, and the milk with enzyme was incubated for 3 days at room temperature (23°C). The residual lactose in each sample was determined as described above ("*Analysis of residual lactose content*") using HPAEC-PAD. A dose/response curve was generated by plotting the measured lactose levels against the dosage of the *B. bifidum* lactase (enzyme #15). See Figure 1.
The data in columns 2 and 4 of Table 2 were used together with the curve to determine the relative activity of each UHT-treated lactase as a percentage of the activity of non-UHT treated *B. bifidum* lactase (enzyme #15). Specifically, for each lactase, the value in column 4 (residual lactose (%) after 72 hrs) was used to determine a corresponding amount of the non-UHT treated *B. bifidum* reference lactase (mg ep/L milk) needed to reach the same residual lactose (%) after incubation at the same time and temperature ("corresponding amount"). This allows calculation of the relative activity of the UHT-treated lactase as a percentage of the activity of non-UHT treated *B. bifidum* reference lactase.
The relative activity may be calculated as the reciprocal of the amount (mg ep/L milk) of UHT-treated lactase (column 2 of Table 2) as a percentage of the reciprocal of the "corresponding amount" (mg ep/L milk) of non-UHT treated *B. bifidum* lactase. In practice, the calculation can be simplified by dividing the "corresponding amount" (mg ep/L milk) of non-UHT treated *B. bifidum* lactase by the amount (mg ep/L milk) of UHT-treated lactase found in column 2 of Table 2 and expressing the result in percent. This measure of the relative activity is shown in column 6 of Table 2, and an example of the calculation is provided in the "Results" section below.
This value is thus a measure of the "survival of enzyme activity after UHT treatment" based on the remaining lactose after UHT treatment and 3 days of incubation at 23°C. The dose/response curve for non-UHT treated *B. bifidum* lactase (SEQ ID NO: 5) (which has also been incubated for 3 days at 23°C) is used to determine the corresponding dosage of non-UHT treated *B. bifidum* lactase.
The lactase enzymes tested included both wild-type enzymes and engineered variants containing one or more substitutions compared to one of the wild-type enzymes to provide improved performance. These substitutions include replacement of a free Cys (homologous to C372 in SEQ ID NO: 5 / enzyme #15), e.g. in enzyme #2 (*Bifidobacterium samirii* lactase with the substitution C372A), introduction of a disulfide, e.g. in enzymes #3 and 8, and replacement of cis-prolines, e.g. in enzymes #4, 5 and 13. An overview of the lactase enzymes, including SEQ ID NOs. for the wild-type sequences, is provided in Table 1 below.

Table 1: Overview of lactase enzymes

| Enzyme number | SEQ ID NO: | Wild-type or variant | Substitutions relative to wild-type sequence |
|---|---|---|---|
| 1 | 1 | *Bifidobacterium samirii* (wild-type) | |
| 2 | | Variant of SEQ ID NO: 1 | C372A |
| 3 | | Variant of SEQ ID NO: 1 | A1073C H1122C |
| 4 | | Variant of SEQ ID NO: 1 | P615T |
| 5 | | Variant of SEQ ID NO: 1 | P65A |
| 6 | | Variant of SEQ ID NO: 1 | C1195G |
| 7 | 2 | *Streptococcus entericus DSM 14446* (wild-type) | |
| 8 | | Variant of SEQ ID NO: 2 | L1064C Y1110C |
| 9 | | Variant of SEQ ID NO: 2 | P52A |
| 10 | | Variant of SEQ ID NO: 2 | G607T |
| 11 | 3 | *Varibaculum sp.* (wild-type) | |
| 12 | 4 | *Urmitella timonensis* (wild-type) | |
| 13 | | Variant of SEQ ID NO: 4 | P620T |
| 14 | | Variant of SEQ ID NO: 4 | G386Q |
| 15 | 5 | *Bifidobacterium bifidum* (wild-type) | |
| 16 | | Variant of SEQ ID NO: 5 | P65A C372A R389A G482A T523N T756K A936S T972C I035C A1129C F1132C K1168C S1195C |
| 17 | | Variant of SEQ ID NO: 5 | P65A C372A R389A G482A T523N P615T T756K A936S T972C I035C A1129C F1132C K1168C S1195C |
| 18 | | Variant of SEQ ID NO: 5 | P65A C372A M386Q R389A G482A T523N T756K A936S T972C I1035C A1129C F1132C K1168C S1195C |
| 19 | | Variant of SEQ ID NO: 5 | C372A M386Q R389A G482A T523N T756K A936S T972C I035C A 1129C F1132C K1168C S1195C |
| 20 | | Variant of SEQ ID NO: 5 | C372A M386Q R389A G482A T523N P615T T756K A936S T972C I035C A1129C F1132C K1168C S1195C |
| 21 | | Variant of SEQ ID NO: 5 | C372A R389A G482A T523N T756K A936S T972C I035C A1129C F1132C K1168C S1195C |
| 22 | | Variant of SEQ ID NO: 5 | C372A |
| 23 | | Variant of SEQ ID NO: 5 | C372A R389A G482A T523N P615T T756K A936S T972C I1035C A1129C F1132C K1168C S1195C |
| 24 | | Variant of SEQ ID NO: 5 | C372A R389A G482A T523N T756K A936S T972C I1035C A1129C F1132C K1168C S1195C C1199S |

(continued)

| Enzyme number | SEQ ID NO: | Wild-type or variant | Substitutions relative to wild-type sequence |
|---|---|---|---|
| 25 | | Variant of SEQ ID NO: 5 | N275S C372A R389A G482A T523N T756K A936S T972C I035C T1076C Y1122C A1129C S1195C C1199S |
| 26 | | Variant of SEQ ID NO: 5 | P65A C372A M386Q R389A G482A T523N P615T T756K A936S T972C I035C A1129C F1132C K1168C S1195C |
| 27 | | Variant of SEQ ID NO: 5 | C372A R389A G482A T523N T756K A936S T972C I1035C A 1129C S1195C |
| 28 | 6 | *Bacillus sp.* S3 (wild-type) | |
| 29 | 7 | *Bifidobacterium aerophilum* (wild-type) | |
| 30 | 8 | *Bifidobacterium mongoliense* (wild-type) | |
| 31 | 9 | *Clostridium nexile CAG:348* (wild-type) | |
| 32 | 10 | *Neobacillus bataviensis* (wild-type) | |
| 33 | 11 | *Neobacillus mesonae* (wild-type) | |
| 34 | 12 | *Neobacillus niacini* (wild-type) | |
| 35 | 13 | *Streptomyces cirratus* (wild-type) | |
| 36 | | Variant of SEQ ID NO: 1 | C372A A1073C H 1122C |
| 37 | | Variant of SEQ ID NO: 1 | C372A C1195G |
| 38 | | Variant of SEQ ID NO: 1 | A1073C H1122C C1195G |
| 39 | | Variant of SEQ ID NO: 1 | C372AA1073C H1122C C1195G |

*Results:*

The results of the assays described above are shown in Tables 2 and 3. Table 2, columns 3 and 4 show the residual lactose at 0.5 hrs and 72 hrs, respectively, after UHT treatment. There is a decrease in the amount of lactose from 0.5 hrs (an average of 4.5 g lactose/100ml, corresponding to 96% of initial lactose) to lower levels of lactose after 72 hrs, where enzymes #1, 3 and 5 are below 0.01% residual lactose, which is needed to claim milk products as "lactose-free" in many countries. The enzyme dose used (mg ep/L milk) is shown in column 2 for each enzyme. If the enzyme dose is increased, the residual lactose after 72 hrs will be lower.

Column 5 shows the pseudo-specific activity (gram lactose converted per mg enzyme protein) after UHT treatment, calculated as ("g/L lactose at 0.5 h" minus "g/L lactose at 72 h")/"mg ep/L", i.e. the value in column 3 minus that in column 4 (although expressed in g/L rather than % lactose) divided by the value in column 2. As an example, using the data for enzyme #12 in Table 2:

$$(45 \text{ g/L lactose} - 12.7 \text{ g/L lactose})/5.5 \text{ mg ep/L} = 5.9 \text{ g lactose/mg ep (in 71.5 hrs)}.$$

This pseudo-specific activity is biased in the sense that it is dose-dependent, since the residual lactose versus enzyme dose is not linear (see Figure 1). As a result, a relatively low enzyme dose gives a higher pseudo-specific activity value compared to a higher enzyme dose.

To have a better comparison of the efficiency of the enzymes, a "Relative activity to unstressed *B. bifidum* lactase" (enzyme #15) has been calculated as described above and is shown in column 6 of Table 2. An example of a calculation for enzyme #12 using Figure 1 is provided here. A dose of 5.5 mg ep of enzyme #12/liter milk was used (Table 2, column 2), resulting in 1.27% residual lactose after 72 hrs (Table 2, column 4). This is shown as the horizontal arrow in Figure 1, which corresponds to 0.39 mg ep of unstressed *B. bifidum* lactase (enzyme #15) as shown in the vertical arrow in Figure 1, i.e., 0.39 mg ep/L milk is the "corresponding amount" in this case.

Using the calculation method described above ("corresponding amount" (mg ep/L milk) of non-UHT treated *B. bifidum* lactase divided by the amount (mg ep/L milk) of UHT-treated lactase), this gives (0.39/5.5 x 100%), i.e., 7.1%, which is the value shown for enzyme #12 in Table 2, column 6. This is a very good measure of the relative activity of the enzyme after UHT treatment compared to the non-UHT treated *B. bifidum* lactase (#15).

Columns 7, 8 and 9 show the calculated enzyme doses needed to reach 1.41% residual lactose (stomach friendly level, 70% reduction of lactose), 0.1% residual lactose (to claim low lactose level in many countries) or 0.01% residual lactose (to claim lactose-free level in many countries) after storage at 23°C for 72 hrs. These numbers are calculated as follows:

The amount of *B. bifidum* lactase (enzyme #15) needed to reach lactose levels of 1.41%, 0.1% and 0.01% is 0.361, 0.934 and 1.56 mg ep/L milk, respectively, determined from the dose-response curve in Fig. 1. These values are used to predict the corresponding amount of a given enzyme needed to reach the same lactose levels by dividing them with the "Relative activity to unstressed *B. bifidum* lactase (%)" value (column 6 in Table 2). For example, for enzyme #12, the "Relative activity to unstressed *B. bifidum* lactase (%)" is 7.1% (0.071) and therefore the calculated (extrapolated) amount of enzyme #12 protein needed to reach the three lactose levels is 0.361/0.071, 0.934/0.071 and 1.56/0.071 mg ep/L milk, which is 5.1, 13.1 and 22.0 mg ep/L milk, respectively, as shown in columns 7, 8 and 9 of Table 2.

Enzyme doses of 5 - 50 mg lactase ep/L milk (typically containing 4.5-5.0% lactose) are typically used in industrial batch lactose-reducing applications, with a tank incubation time in the range of 8 - 24 h at 4 - 10°C to reach lactose levels of 0.01 - 0.1% lactose (% = g lactose /100ml milk). Thus, the top candidates in Table 2 are highly relevant for lactose-free applications (0.01% lactose), whereas other sequences with a lower relative activity may be more relevant for low-lactose applications rather than "lactose-free" applications.

Table 3, columns 2 and 3, show residual activity of the enzymes at 0.5 hrs and 72 hrs, respectively, after UHT treatment. It may be seen that the enzymes exhibit various degrees of residual activity, and surprisingly, that most of them have an increased residual activity after 72 hrs compared to 0.5 hrs, with the average increase for the listed enzymes being more than 3-fold.

The reason for a relatively low correlation between % residual activity (Table 3) and measured residual lactose levels in Table 2 is believed to be the different specific activities of each enzyme. The ability of an enzyme to hydrolyze lactose after UHT treatment is influenced by the enzyme's efficiency to convert lactose (specific activity before UHT treatment) as well as enzyme activity after UHT treatment (% residual activity).

Table 2: Residual lactose and relative lactase activity after UHT treatment

| Enzyme number | mg ep/L milk | Residual lactose (%): | | Gram lactose per mg ep | Relative activity (%) to unstressed *B. bifidum* lactase | Extrapolated enzyme protein (mg) to reach indicated residual lactose % in milk: | | |
|---|---|---|---|---|---|---|---|---|
| | | after 0.5 hr | after 72 hr | | | 1.41% | 0.1% | 0.01% |
| 1 | 13.4 | 4.4 | 0.042 | 3.3 | 8.3 | 4.3 | 11.3 | 18.8 |
| 2 | 10.6 | 4.4 | 0.007 | 4.2 | 15.9 | 2.3 | 5.9 | 9.8 |
| 3 | 9.0 | 4.6 | 0.020 | 5.0 | 13.7 | 2.6 | 6.8 | 11.4 |
| 4 | 12.9 | 4.6 | 0.019 | 3.5 | 9.9 | 3.7 | 9.5 | 15.8 |
| 5 | 13.1 | 4.6 | 0.020 | 3.5 | 9.5 | 3.8 | 9.8 | 16.4 |
| 6 | 6.4 | 4.6 | 0.54 | 6.3 | 9.2 | 3.9 | 10.1 | 16.9 |
| 7 | 12.7 | 4.6 | 1.99 | 2.1 | 2.0 | 17.7 | 45.8 | 76.6 |
| 8 | 9.4 | 4.2 | 0.059 | 4.5 | 11.1 | 3.2 | 8.4 | 14.0 |
| 9 | 11.9 | 4.4 | 0.092 | 3.6 | 8.0 | 4.5 | 11.7 | 19.6 |
| 10 | 17.4 | 4.3 | 0.20 | 2.4 | 4.5 | 8.0 | 20.7 | 34.6 |
| 11 | 12.7 | 4.5 | 0.86 | 2.9 | 3.9 | 9.4 | 24.2 | 40.5 |

(continued)

| Enzyme number | mg ep/L milk | Residual lactose (%): | | Gram lactose per mg ep | Relative activity (%) to unstressed *B. bifidum* lactase | Extrapolated enzyme protein (mg) to reach indicated residual lactose % in milk: | | |
|---|---|---|---|---|---|---|---|---|
| | | after 0.5 hr | after 72 hr | | | 1.41% | 0.1% | 0.01% |
| 12 | 5.5 | 4.5 | 1.27 | 5.9 | 7.1 | 5.1 | 13.1 | 22.0 |
| 13 | 12.3 | 4.5 | 0.009 | 3.6 | 11.6 | 3.1 | 8.0 | 13.4 |
| 14 | 16.2 | 4.2 | 0.006 | 2.6 | 10.9 | 3.3 | 8.6 | 14.3 |
| 15 | 12.7 | 4.6 | 2.22 | 1.9 | 1.8 | 20.3 | 52.5 | 87.8 |
| 16 | 12.7 | 4.6 | 0.36 | 3.3 | 5.3 | 6.9 | 17.8 | 29.7 |
| 17 | 12.7 | 4.6 | 0.71 | 3.1 | 4.2 | 8.6 | 22.3 | 37.3 |
| 18 | 12.7 | 4.6 | 0.85 | 3.0 | 3.9 | 9.3 | 24.0 | 40.2 |
| 19 | 12.7 | 4.6 | 0.95 | 2.9 | 3.7 | 9.7 | 25.2 | 42.1 |
| 20 | 12.7 | 4.6 | 1.03 | 2.8 | 3.6 | 10.1 | 26.1 | 43.7 |
| 21 | 12.7 | 4.6 | 1.24 | 2.6 | 3.2 | 11.5 | 29.6 | 49.6 |
| 22 | 12.8 | 4.6 | 1.42 | 2.4 | 2.8 | 12.9 | 33.5 | 56.1 |
| 23 | 12.7 | 4.6 | 1.60 | 2.4 | 2.5 | 14.2 | 36.8 | 61.5 |
| 24 | 12.7 | 4.6 | 1.70 | 2.3 | 2.4 | 15.0 | 38.9 | 65.0 |
| 25 | 12.7 | 4.6 | 1.71 | 2.3 | 2.4 | 15.1 | 39.0 | 65.3 |
| 26 | 12.7 | 4.6 | 1.75 | 2.3 | 2.3 | 15.4 | 40.0 | 66.9 |
| 27 | 12.7 | 4.6 | 1.97 | 2.1 | 2.1 | 17.5 | 45.4 | 75.9 |
| 28 | 22.2 | 4.0 | 0.60 | 1.5 | 2.5 | 14.2 | 36.8 | 61.5 |
| 29 | 12.7 | 4.6 | 3.19 | 1.1 | 0.9 | 38.6 | 99.9 | 167.1 |
| 30 | 12.8 | 4.6 | 3.49 | 0.9 | 0.7 | 50.2 | 130.0 | 217.4 |
| 31 | 6.6 | 4.6 | 2.68 | 2.9 | 2.6 | 14.1 | 36.5 | 61.0 |
| 32 | 12.4 | 4.5 | 1.66 | 2.3 | 2.5 | 14.3 | 37.1 | 62.0 |
| 33 | 11.2 | 4.5 | 2.14 | 2.1 | 2.1 | 17.1 | 44.2 | 73.9 |
| 34 | 31.6 | 4.6 | 2.05 | 0.8 | 0.8 | 45.7 | 118.3 | 197.9 |
| 35 | 11.0 | 4.2 | 0.87 | 3.0 | 4.4 | 8.1 | 21.0 | 35.2 |
| 36 | 14.7 | 4.3 | 0.031 | 2.9 | 8.0 | 4.5 | 11.7 | 19.5 |
| 37 | 16.9 | 4.3 | 0.012 | 2.5 | 8.1 | 4.5 | 11.5 | 19.3 |
| 38 | 16.5 | 4.2 | 0.018 | 2.5 | 7.8 | 4.6 | 11.9 | 20.0 |
| 39 | 15.7 | 4.5 | 0.0078 | 2.9 | 10.7 | 3.4 | 8.7 | 14.6 |

Table 3: Residual lactase activity after UHT treatment

| Enzyme number | Residual activity (%) after 0.5 h (RA 72h) | Residual activity (%) after 72 h (RA 0.5h) | (RA 72h) / (RA 0.5h) |
|---|---|---|---|
| 1 | 2.1 | 5.1 | 2.4 |
| 2 | 11 | 15 | 1.5 |

(continued)

| Enzyme number | Residual activity (%) after 0.5 h (RA 72h) | Residual activity (%) after 72 h (RA 0.5h) | (RA 72h) / (RA 0.5h) |
|---|---|---|---|
| 3 | 2.1 | 13 | 6.2 |
| 4 | 2.5 | 8.0 | 3.2 |
| 5 | 1.2 | 5.7 | 4.6 |
| 6 | 3.3 | 11 | 3.2 |
| 7 | 18 | 37 | 2.0 |
| 8 | 13 | 25 | 1.9 |
| 9 | 0.8 | 3.3 | 4.2 |
| 10 | 9.7 | 14 | 1.5 |
| 11 | 2.4 | 10.8 | 4.4 |
| 12 | 1.3 | 4.2 | 3.2 |
| 13 | 17 | 48 | 2.9 |
| 14 | 5.4 | 17 | 3.1 |
| 15 | 0.2 | 0.7 | 4.3 |
| 16 | 3.0 | 9.9 | 3.3 |
| 17 | 2.7 | 7.6 | 2.8 |
| 18 | 4.2 | 11.9 | 2.9 |
| 19 | 3.7 | 9.5 | 2.5 |
| 20 | 2.6 | 7.7 | 2.9 |
| 21 | 2.9 | 6.4 | 2.2 |
| 22 | 1.0 | 1.2 | 1.2 |
| 23 | 2.5 | 6.3 | 2.5 |
| 24 | 4.7 | 8.4 | 1.8 |
| 25 | 2.7 | 7.0 | 2.6 |
| 26 | 3.5 | 8.9 | 2.5 |
| 27 | 1.0 | 3.0 | 3.0 |
| 28 | 23 | 34 | 1.5 |
| 29 | 0.2 | 1.8 | 9.1 |
| 30 | 0.1 | 0.8 | 7.4 |
| 31 | 0.4 | 8.0 | 19.4 |
| 32 | 12 | 14 | 1.1 |
| 33 | 12 | 10 | 0.8 |
| 34 | 3.3 | 8.1 | 2.5 |
| 35 | 60 | 68 | 1.1 |
| 36 | 14.0 | 19.0 | 1.4 |
| 37 | 23.7 | 28.9 | 1.2 |
| 38 | 6.7 | 17.4 | 2.6 |
| 39 | 21.1 | 28.9 | 1.4 |

**Example 2**

*Determination of Tm using thermal shift assay at pH 6 and 7*

The thermal shift assay (TSA) measures the melting temperature of a protein (Tm), which is the temperature at which there is 50% denaturation. Protein denaturation is monitored via an increase in fluorescence of SYPRO™ Orange dye, which binds to hydrophobic residues that become exposed as the target protein unfolds.

The purified samples were diluted to 0.24 mg/ml in Milli-Q water. The thermal shift assay mix was prepared by diluting SYPRO™ Orange (Invitrogen/ThermoFisher # S6650) 200-fold into the desired pH buffer (100 mM succinic acid, 100 mM HEPES, 100 mM glycine, 150 mM KCl, 1 mM CaCl2, 0.01% Triton-X 100, adjusted to pH 6 or 7). 10 µl of diluted sample was added to 20 µl TSA mix in a 96-well (LightCycler® 480 multi-well plate, Roche # 04729692001). After sealing with optical tape (Roche, # 04729757001), the plate was heated from 25°C to 95°C (temperature ramp: 3.2°C/minute) in a LightCycler® 480 II Real-Time PCR machine (Roche) and the fluorescence was continuously measured (excision/emission wavelengths: 465/510 nm). Tm is identified by plotting the first derivative of the fluorescence as a function of temperature (dF/dT) and determining the temperature with maximal dF/dt.

The determined Tm values are shown in Table 4.

Table 4: Melting temperature Tm (°C) as determined by TSA at pH 6 and 7

| Enzyme number | Donor | Substitutions | Tm @ pH 6 | Tm @ pH 7 |
|---|---|---|---|---|
| 1 | *Bifidobacterium samirii* | - | 56.2 | 57.2 |
| 12 | *Urmitella timonensis* | - | 53.4 | 55.0 |
| 7 | *Streptococcus entericus* | - | 53.0 | 54.1 |
| 15 | *Bifidobacterium bifidum* | - | 55.8 | 56.6 |
| 35 | *Streptomyces cirratus* | - | 55.5 | 55.8 |
| 28 | *Bacillus sp. S3* | - | 66.6 | 66.7 |
| 2 | *Bifidobacterium samirii* | C372A | 56.4 | 57.0 |
| 3 | *Bifidobacterium samirii* | A1073C H1122C | 57.0 | 57.0 |
| 13 | *Urmitella timonensis* | P620T | 50.0 | 50.6 |
| 14 | *Urmitella timonensis* | G386Q | 50.9 | 50.6 |
| 11 | *Varibaculum sp.* | | 62.6 | 63.1 |
| 8 | *Streptococcus entericus* | L1064C Y1110C | 52.2 | 53.9 |
| 6 | *Bifidobacterium samirii* | C1195G | 57.7 | 58.1 |

**Example 3**

*Determination of lactase temperature optimum*

The temperature profile from 35-75°C was determined for five lactase enzymes using Method 2 above.

The results can be seen below in Table 5, which shows the relative activity (percentage of maximum activity) for the five enzymes at different temperatures. The table shows that there is a temperature optimum of about 43.0°C, 47.5°C, 38.9°C, 43°C and <35°C for enzymes number 1, 12, 7, 15 and 11, respectively (indicated by "100"). The table also shows the temperature at which 50% activity remains (after the temperature optimum), which is approximately 55°C, 55°C, 46°C, 58°C and 52°C, respectively (evaluated from Table 5, between the two numbers with an asterisk (*)).

The temperature profile is typically related to the temperature dependence of the lactose hydrolysis and the enzyme's unfolding rate. If unfolding of the enzyme is fully reversible, then the decline in activity after the optimum should correlate with the amount of unfolded enzyme, and the "melting temperature" (Tm, where half of the enzyme is unfolded) should be close to the value for 50% residual activity as determined from Table 5. However, typically the unfolding is irreversible and there is also a temperature dependence of the lactose hydrolysis rate, both of which have an impact on the decline in activity.

Since the Tm values for the enzymes in 1, 12, 7, 15 and 11 have been determined to be approximately 57°C, 55°C, 54°C, 56°C and 63°C, respectively (see Table 4 in Example 2), there is a close match between Tm (Table 4) and 50% residual activity (Table 5) for enzymes number 1, 12 and 15. This suggests that these enzymes have a pronounced amount of refolding in the assay.

The minor amount of relative activity (2-5%) at higher temperatures (>70°C), which seems rather constant, is believed to be due to the ramp up time from room temperature to >70°C (ramp up time is expected to be below 1 min).

Table 5: Relative activity of lactase enzymes at different temperatures

| Temp. (°C) | Enz no. 1 (SEQ ID NO: 1) | Enz no. 12 (SEQ ID NO: 4) | Enz no. 7 (SEQ ID NO: 2) | Enz no. 15 (SEQ ID NO: 5) | Enz no. 11 (SEQ ID NO: 3) |
|---|---|---|---|---|---|
| 35 | 89 | 92 | 92 | 99 | **100** |
| 36.1 | 93 | 92 | 98 | 91 | 98 |
| 38.9 | 96 | 98 | **100** | 97 | 93 |
| 43 | **100** | 97 | 97* | **100** | 71 |
| 47.5 | 95 | **100** | 28* | 99 | 57* |
| 52.5 | 77* | 79* | 17 | 93 | 48* |
| 57.5 | 31* | 29* | 8 | 68* | 29 |
| 62.3 | 6 | 8 | 4 | 19* | 14 |
| 67 | 4 | 5 | 4 | 6 | 7 |
| 71.1 | 3 | 4 | 4 | 5 | 4 |
| 73.9 | 3 | 3 | 4 | 5 | 3 |
| 75 | 2 | 3 | 3 | 4 | 4 |

**Claims**

1. A milk-based product comprising an enzyme having lactase activity, wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

2. The milk-based product of claim 1, wherein the enzyme having lactase activity comprises an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

3. The milk-based product of claim 2, wherein the enzyme having lactase activity comprises an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4.

4. The milk-based product of claim 3, wherein the enzyme having lactase activity is selected from the group consisting of:

   a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;
   b) a variant of SEQ ID NO: 2 comprising at least one substitution selected from the group consisting of P52A, G607T, L1064C and Y1110C, for example two or more of said substitutions, wherein numbering is based on SEQ ID NO: 2; and
   c) a variant of SEQ ID NO: 4 comprising the substitution G386Q and/or P620T, wherein numbering is based on SEQ ID NO: 4.

5. The milk-based product of any of claims 1-4, wherein the enzyme having lactase activity has a length of at the most about 1500 amino acids, such as at the most about 1400 amino acids or at the most about 1350 amino acids, for example 850-1500 amino acids, preferably 850-1400 amino acids, such as 850-1350 amino acids.

6. The milk-based product of any of claims 1-5, wherein the product has been heat-treated and has a lactose content of at most 0.2% (w/w).

7. The milk-based product of any of claims 1-6, wherein the product is UHT milk, ESL milk or ultra-pasteurized milk.

8. An enzyme having lactase activity, wherein the enzyme is selected from the group consisting of:

a) a polypeptide having an amino acid sequence which is at least 75%, such as at least 80%, at least 85%, at least 90%, at least 95%, at least 98%, at least 99%, at least 99.5% or 100% identical to SEQ ID NO: 1, wherein the polypeptide has a length of at the most about 1500 amino acids, such as at the most about 1400 amino acids or at the most about 1350 amino acids;
b) a polypeptide having an amino acid sequence which is at least 75%, such as at least 80%, at least 85%, at least 90%, at least 95%, at least 98%, at least 99%, at least 99.5% or 100% identical to SEQ ID NO: 2, wherein the polypeptide has a length of at the most about 1500 amino acids, such as at the most about 1400 amino acids or at the most about 1350 amino acids;
c) a polypeptide having an amino acid sequence which is at least 75%, such as at least 80%, at least 85%, at least 90%, at least 95%, at least 98%, at least 99%, at least 99.5% or 100% identical to SEQ ID NO: 3, wherein the polypeptide has a length of at the most about 1500 amino acids, such as at the most about 1400 amino acids or at the most about 1350 amino acids;
d) a polypeptide having an amino acid sequence which is at least 75%, such as at least 80%, at least 85%, at least 90%, at least 95%, at least 98%, at least 99%, at least 99.5% or 100% identical to SEQ ID NO: 4, wherein the polypeptide has a length of at the most about 1500 amino acids, such as at the most about 1400 amino acids or at the most about 1350 amino acids;
e) a polypeptide having an amino acid sequence which is at least 75%, such as at least 80%, at least 85%, at least 90%, at least 95%, at least 98%, at least 99%, at least 99.5% or 100% identical to SEQ ID NO: 6, wherein the polypeptide has a length of at the most about 1500 amino acids, such as at the most about 1400 amino acids or at the most about 1350 amino acids;
f) a polypeptide having an amino acid sequence which is at least 75%, such as at least 80%, at least 80%, at least 85%, at least 90%, at least 95%, at least 98%, at least 99%, at least 99.5% or 100% identical to SEQ ID NO: 7, wherein the polypeptide has a length of at the most about 1500 amino acids, such as at the most about 1400 amino acids or at the most about 1350 amino acids;
g) a polypeptide having an amino acid sequence which is at least 75%, such as at least 80%, at least 85%, at least 90%, at least 95%, at least 98%, at least 99%, at least 99.5% or 100% identical to SEQ ID NO: 8, wherein the polypeptide has a length of at the most about 1500 amino acids, such as at the most about 1400 amino acids or at the most about 1350 amino acids;
h) a polypeptide having an amino acid sequence which is at least 75%, such as at least 80%, at least 80%, at least 85%, at least 90%, at least 95%, at least 98%, at least 99%, at least 99.5% or 100% identical to SEQ ID NO: 9, wherein the polypeptide has a length of at the most about 1500 amino acids, such as at the most about 1400 amino acids or at the most about 1350 amino acids;
i) a polypeptide having an amino acid sequence which is at least 75%, such as at least 80%, at least 80%, at least 85%, at least 90%, at least 95%, at least 98%, at least 99%, at least 99.5% or 100% identical to SEQ ID NO: 10, wherein the polypeptide has a length of at the most about 1500 amino acids, such as at the most about 1400 amino acids or at the most about 1350 amino acids;
j) a polypeptide having an amino acid sequence which is at least 75%, such as at least 80%, at least 80%, at least 85%, at least 90%, at least 95%, at least 98%, at least 99%, at least 99.5% or 100% identical to SEQ ID NO: 11, wherein the polypeptide has a length of at the most about 1500 amino acids, such as at the most about 1400 amino acids or at the most about 1350 amino acids;
k) a polypeptide having an amino acid sequence which is at least 75%, such as at least 80%, at least 80%, at least 85%, at least 90%, at least 95%, at least 98%, at least 99%, at least 99.5% or 100% identical to SEQ ID NO: 12, wherein the polypeptide has a length of at the most about 1500 amino acids, such as at the most about 1400 amino acids or at the most about 1350 amino acids; and
l) a polypeptide having an amino acid sequence which is at least 75%, such as at least 80%, at least 85%, at least 90%, at least 95%, at least 98%, at least 99%, at least 99.5% or 100% identical to SEQ ID NO: 13, wherein the polypeptide has a length of at the most about 1750 amino acids, such as at the most about 1700 amino acids.

9. The enzyme of claim 8, wherein the enzyme has lactase activity and is selected from the group consisting of:

   a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;
   b) a variant of SEQ ID NO: 2 comprising at least one substitution selected from the group consisting of P52A, G607T, L1064C and Y1110C, for example two or more of said substitutions, wherein numbering is based on SEQ ID NO: 2; and
   c) a variant of SEQ ID NO: 4 comprising the substitution G386Q and/or P620T, wherein numbering is based on SEQ ID NO: 4.

10. A method of producing a lactose-reduced, heat-treated, milk-based product, the method comprising:

    a) adding an enzyme having lactase activity to a milk-based substrate comprising at least 2% lactose (w/w), wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13,
    b) after addition of the enzyme, performing a heat treatment of the milk-based substrate by holding said milk-based substrate at a holding temperature of at least 120°C for a holding time of at least 1 second followed by cooling to produce a heat-treated milk-based product, and
    c) storing the heat-treated milk-based product for at least about 24 hours, preferably at least 2 days, such as at least 3 days, preferably at least 4 days at a temperature of at most 40°C,

    wherein after step b) but before step c) the lactose content in the milk-based product is at least 0.5% (w/w), preferably at least 1% (w/w), and wherein after step c) the lactose content in the milk-based product is at most 0.2% (w/w), and preferably wherein, after step b) but before step c), the lactose content in the milk-based product is at least 0.5% (w/w), preferably at least 1% (w/w).

11. The method of claim 10, wherein the enzyme having lactase activity comprises an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to SEQ ID NO: 1, SEQ ID NO: 2, SEQ ID NO: 3 or SEQ ID NO: 4.

12. The method of claim 11, wherein the enzyme having lactase activity is selected from the group consisting of:

    a) a variant of SEQ ID NO: 1 comprising at least one substitution selected from the group consisting of P65A, C372A, P615T, A1073C, H1122C and C1195G, for example two, three, four or more of said substitutions, wherein numbering is based on SEQ ID NO: 1;
    b) a variant of SEQ ID NO: 2 comprising at least one substitution selected from the group consisting of P52A, G607T, L1064C and Y1110C, for example two or more of said substitutions, wherein numbering is based on SEQ ID NO: 2; and
    c) a variant of SEQ ID NO: 4 comprising the substitution G386Q and/or P620T, wherein numbering is based on SEQ ID NO: 4.

13. The method of any of claims 10-12, wherein step b) is performed immediately after step a) without a dedicated incubation step between step a) and step b).

14. A method of producing a lactose-reduced milk product, the method comprising adding an enzyme having lactase activity to a milk-based substrate comprising at least 2% lactose (w/w), and subjecting the milk-based substrate comprising the enzyme to a heat treatment at a temperature of at least 120°C for a holding time of at least 1 second, wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

15. Use of an enzyme having lactase activity for producing a lactose-reduced, heat-treated, milk-based product in a method wherein a milk-based substrate comprising at least 2% lactose (w/w) to which the enzyme has been added is subjected to a heat treatment at a temperature of at least 120°C for a holding time of at least 1 second, wherein the enzyme having lactase activity comprises an amino acid sequence having at least 75% sequence identity to SEQ ID NO: 1, SEQ ID NO: 2, SEQ ID NO: 3, SEQ ID NO: 4, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

# Figure 1

Europäisches
Patentamt

European
Patent Office

Office européen
des brevets

# EUROPEAN SEARCH REPORT

**Application Number**

EP 22 21 5776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/210821 A1 (NOVOZYMES AS [DK]) 22 November 2018 (2018-11-22) | 1-3,6,7, 10,11, 13-15 | INV. C12N9/38 A23C3/03 |
| A | * page 22, item 1(a); page 24, item 24; page 27, item 37; page 11, line 6 - line 9; claim 7; table 1; sequences 1,13 * & DATABASE Geneseq [Online]<br><br>10 January 2019 (2019-01-10), "Bifidobacterium bifidum beta-galactosidase fragment, SEQ ID 1.", retrieved from EBI accession no. GSP:BFV90082 Database accession no. BFV90082 * sequence *<br>————— | 4,5,8,9, 12 | A23C3/037 A23C9/12 |
| A | DATABASE UniProt [Online]<br><br>8 May 2019 (2019-05-08), "SubName: Full=Beta-galactosidase BbgIII {ECO:0000313¦EMBL:RSX57731.1};", XP002809305, retrieved from EBI accession no. UNIPROT:A0A430FVJ2 Database accession no. A0A430FVJ2 * sequence *<br>————— | 1-15 | |

**TECHNICAL FIELDS SEARCHED (IPC)**

C12N
A23C

The present search report has been drawn up for all claims

| Place of search | Date of completion of the search | Examiner |
|---|---|---|
| The Hague | 16 May 2023 | Du, Mingliu |

Europäisches
Patentamt

European
Patent Office

Office européen
des brevets

**Application Number**

**EP 22 21 5776**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

37

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-15(partially)

     enzyme having lactase activity comprising an amino acid sequence having at least 75% sequence identity to SEQ ID NO:1, a milk-based product comprising the enzyme, a method of producing a milk-based product with the enzyme and use of the enzyme for producing milk-based product
     ---

2. claims: 1-15(partially)

     enzyme having lactase activity comprising an amino acid sequence having at least 75% sequence identity to SEQ ID NO:2, a milk-based product comprising the enzyme, a method of producing a milk-based product with the enzyme and use of the enzyme for producing milk-based product
     ---

3. claims: 1-3, 5-8, 10, 11, 13-15(all partially)

     enzyme having lactase activity comprising an amino acid sequence having at least 75% sequence identity to SEQ ID NO:3, a milk-based product comprising the enzyme, a method of producing a milk-based product with the enzyme and use of the enzyme for producing milk-based product
     ---

4. claims: 1-15(partially)

     enzyme having lactase activity comprising an amino acid sequence having at least 75% sequence identity to SEQ ID NO:4, a milk-based product comprising the enzyme, a method of producing a milk-based product with the enzyme and use of the enzyme for producing milk-based product
     ---

5. claims: 1, 2, 5-8, 10, 13-15(all partially)

     enzyme having lactase activity comprising an amino acid sequence having at least 75% sequence identity to SEQ ID NO:6, a milk-based product comprising the enzyme, a method of producing a milk-based product with the enzyme and use of the enzyme for producing milk-based product
     ---

6. claims: 1, 2, 5-8, 10, 13-15(all partially)

     enzyme having lactase activity comprising an amino acid sequence having at least 75% sequence identity to SEQ ID NO:7, a milk-based product comprising the enzyme, a method of producing a milk-based product with the enzyme and use of

page 1 of 3

Europäisches
Patentamt

European
Patent Office

Office européen
des brevets

**LACK OF UNITY OF INVENTION**
**SHEET B**

Application Number

EP 22 21 5776

The Search Division considers that the present European patent application does not comply with the requirements of unity of invention and relates to several inventions or groups of inventions, namely:

```
     the enzyme for producing milk-based product
                      ---


7. claims: 1, 2, 5-8, 10, 13-15(all partially)

     enzyme having lactase activity comprising an amino acid
     sequence having at least 75% sequence identity to SEQ ID
     NO:8, a milk-based product comprising the enzyme, a method
     of producing a milk-based product with the enzyme and use of
     the enzyme for producing milk-based product
                      ---


8. claims: 1, 2, 5-8, 10, 13-15(all partially)

     enzyme having lactase activity comprising an amino acid
     sequence having at least 75% sequence identity to SEQ ID
     NO:9, a milk-based product comprising the enzyme, a method
     of producing a milk-based product with the enzyme and use of
     the enzyme for producing milk-based product
                      ---


9. claims: 1, 2, 5-8, 10, 13-15(all partially)

     enzyme having lactase activity comprising an amino acid
     sequence having at least 75% sequence identity to SEQ ID
     NO:10, a milk-based product comprising the enzyme, a method
     of producing a milk-based product with the enzyme and use of
     the enzyme for producing milk-based product
                      ---


10. claims: 1, 2, 5-8, 10, 13-15(all partially)

     enzyme having lactase activity comprising an amino acid
     sequence having at least 75% sequence identity to SEQ ID
     NO:11, a milk-based product comprising the enzyme, a method
     of producing a milk-based product with the enzyme and use of
     the enzyme for producing milk-based product
                      ---


11. claims: 1, 2, 5-8, 10, 13-15(all partially)

     enzyme having lactase activity comprising an amino acid
     sequence having at least 75% sequence identity to SEQ ID
     NO:12, a milk-based product comprising the enzyme, a method
     of producing a milk-based product with the enzyme and use of
     the enzyme for producing milk-based product
                      ---


12. claims: 1, 2, 5-8, 10, 13-15(all partially)

     enzyme having lactase activity comprising an amino acid
```

page 2 of 3

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
sequence having at least 75% sequence identity to SEQ ID
NO:13, a milk-based product comprising the enzyme, a method
of producing a milk-based product with the enzyme and use of
the enzyme for producing milk-based product
                          ---
```

**EP 4 389 886 A1**

## ANNEX TO THE EUROPEAN SEARCH REPORT
## ON EUROPEAN PATENT APPLICATION NO.

EP 22 21 5776

16-05-2023

| Patent document cited in search report | | Publication date | Patent family member(s) | | Publication date |
|---|---|---|---|---|---|
| WO 2018210821 | A1 | 22-11-2018 | AU | 2018267948 A1 | 14-11-2019 |
| | | | AU | 2018269327 A1 | 21-11-2019 |
| | | | BR | 112019024124 A2 | 02-06-2020 |
| | | | BR | 112019024134 A2 | 23-06-2020 |
| | | | CN | 110621163 A | 27-12-2019 |
| | | | CN | 110868865 A | 06-03-2020 |
| | | | EP | 3624593 A1 | 25-03-2020 |
| | | | EP | 3624594 A1 | 25-03-2020 |
| | | | US | 2020113200 A1 | 16-04-2020 |
| | | | US | 2020232004 A1 | 23-07-2020 |
| | | | US | 2022232843 A1 | 28-07-2022 |
| | | | WO | 2018210820 A1 | 22-11-2018 |
| | | | WO | 2018210821 A1 | 22-11-2018 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009071539 A
- WO 2018189238 A, Chr. Hansen
- WO 2020176734 A
- EP 21216998
- WO 9517413 A
- WO 9522625 A

- US 5223409 A
- WO 9206204 A
- WO 09071539 A1
- WO 2003095658 A
- WO 9943835 A

### Non-patent literature cited in the description

- **DEETH.** Optimum Thermal Processing for Extended Shelf-Life (ESL) Milk. *Foods,* 2017, vol. 6 (11), 102
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **JUMPER et al.** Highly accurate protein structure prediction with AlphaFold. *Nature,* 2021, vol. 596, 583-589

- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOW-MAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **HORTON, R.M. ; HUNT, H.D. ; HO, S.N. ; PULLEN, J.K. ; PEASE, L.R.** Engineering hybrid genes without the use of restriction enzymes, gene splicing by overlap extension. *Gene,* 1989, vol. 77, 61-68
- **LEEUWEN S ; KUIPERS B ; DIJKHUIZEN L ; KA-MERLING J.** Comparative structural characterization of 7 commercial galacto-oligosaccharide (GOS) products. *Carbohydrate Research,* 2016, vol. 425, 48-58